# EUROPEAN PATENT APPLICATION

(11) **EP 4 681 780 A1**
(43) Date of publication of application: **21.01.2026**
(21) Application number: 24306214.8
(22) Date of filing: 18.07.2024
(51) Int. Cl.: A61P 35/00, A61K 38/00, A61K 39/00, C07K 14/55, C07K 14/715, C07K 16/28, C12N 15/62

(54) **IMMUNOCYTOKINE FOR CANCER TREATMENT**

(71) Applicant: Egle Therapeutics, 75014 Paris (FR)
(72) Inventor: The designation of the inventor has not yet been filed
(74) Representative: Ipsilon

(57) **Abstract**

The present invention relates to a fusion protein comprising an anti-CCR8 antibody, or a fragment thereof, and at least one IL-2 variant which is a regulatory T cell (Treg)-specific IL-2 receptor antagonist; wherein the N-terminus of the at least one IL-2 variant is fused to the C-terminus of the antibody heavy chain. It also relates to a pharmaceutical composition comprising at least such a fusion protein as well as to the implementation of said fusion protein or composition for the treatment of cancer.

## Description

### FIELD OF THE INVENTION

The invention pertains to the field of immunotherapy. The invention relates to immunocytokines comprising an anti-CCR8 antibody, or a fragment thereof, fused to at least one regulatory T cell (Treg)-specific IL-2 receptor antagonist and their use to selectively deplete tumor-infiltrating Tregs and in particular tumor-associated Tregs, in particular for treating cancer.

### BACKGROUND OF THE INVENTION

Regulatory T cells (Tregs) are a key component of the microenvironment of several tumour types that prevent effector T cells and NK cells from mediating an immune response. Eliminating Treg cells from the tumour microenvironment restores immune responses and contribute to the elimination of the tumour (Exemplified by Curiel et al., Nat. Med., 2004, 10, 942-949; Hiraoka et al., Clin. Cancer Res., 2006, 12, 5423-5434; Stirm et al., Journal for ImmunoTherapy of Cancer 2023;11:e006263; and reviewed by Shan et al., Trends in Cancer, November 2022, Vol. 8, No. 11). Given the role for Tregs in protection against autoimmune diseases, immune intervention aiming at depleting Treg cells must selectively deplete only tumor-infiltrating Tregs (Fontenot et al., Nat. Immunol., 2003, 4, 330-336).

Treg cell survival is under the dependency of interleukin-2 (IL-2), which is mainly produced by effector T cells and NK cells, and signals in Treg cells via the high affinity IL-2 receptor (IL-2R) complex composed of alpha (IL-2-RA or CD25), beta (IL-2RB or CD122) and gamma (IL-2RG or Gamma c or CD132) chains. The alpha chain binds IL-2 with low affinity and does not participate in signaling. The combination of the beta and gamma chains forms an intermediate affinity receptor expressed on the effector T cells (conventional T (Tconv) cells (CD4+Foxp3-) and CD8+ T cells) and NK cells responsible for the cell-mediated immune responses; and all three receptor chains form a high affinity receptor expressed constitutively on regulatory T cells (CD4+Foxp3+) which have immunosuppressive function, and shortly or transiently by activated effector T cells and NK cells.

Various IL-2 variants (IL-2 muteins) have been generated to modulate IL-2 biological activity for cancer treatment (Review in Leon et al., Seminars in Oncology, 2018, 45, 95-104). The no-alpha mutein is an agonist of IL-2R signaling with a reduced ability to expand Treg in vivo (Carmenate et al., J. Immul., 2013, 190, 6230-6238 ; Rojas et al., J. Mol. Recognit., 2015, 25, 261-268). The plus-beta mutein or IL-2 superkine is an agonist of IL-2R signaling with preferential in vivo expansion of CD8+ T cells and NK cells, with some effects on Tregs (Levin et al., Nature, 2012, 484, 529-533). The no-gamma mutein is an antagonist of IL-2R signaling with some preferential affinity for Tregs (Carmenate et al., J. Immunol., 2018, 200, 3475-3484). In mice, the no-alpha and plus-beta muteins have higher antitumor activity and lower toxicity than wild-type IL-2. The no-gamma mutein has antitumoral effect similar to anti-CD25 monoclonal antibodies in mice. Other antagonists of IL-2R signaling having the ability to inhibit Treg survival by depriving them from wild-type IL-2 signaling while leaving active IL-2 signaling in CD8+ T cells are disclosed in WO2020/201095 (IL-2V1; IL2-V4; IL-2V5; IL-2V6).

C-C motif chemokine receptor 8 (CCR8) is a seven transmembrane G-coupled protein that is highly expressed by the Tregs within tumors but not those in the blood or other organs (Weaver et al. Oncoimmunology. 2022 Nov 4;11(1):2141007). It is also absent on activated T cells. It was reported that CCL1 (C-C chemokine ligand 1), CCL4 (C-C chemokine ligand 4), CCL16 (C-C chemokine ligand 16), CCL17 (C-C chemokine ligand 17), CCL18 (C-C chemokine ligand 18), and mouse CCL8 (C-C chemokine ligand 8) can bind to CCR8. However, CCR8 is the only receptor for chemokine CCL1 (Korbecki et al. Int. J. Mol. Sci. 2020, 21, 7619). These chemokine ligands serve as an attractant signal which leads to Tregs expressing CCR8 undergoing chemotaxis to migrate towards them. CCL1 is produced by tumor associated macrophages and cancer associated fibroblasts, which can therefore attract CCR8-expressing regulatory T cells to the tumor microenvironment, leading to suppression of anti-tumor immunity. In addition, CCL1 expression in lymph nodes facilitates migration and metastasis of CCR8 expressing cancer cells (Das et al. J Exp Med . 2013 Jul 29;210(8): 1509-28).

In breast cancer patients, CCR8 expression is highly enriched on tumor-associated Tregs compared to circulating peripheral Tregs (Plitas et al., Immunity. 2016 Nov 15;45(5): 1122-1134). Additionally, increased expression of CCR8 has been observed in several types of cancer including breast, colorectal, and melanoma and is associated with poor clinical prognosis (Magnuson et al. Proc Natl Acad Sci U S A. 2018 Nov 6; 115(45):E10672-E10681). The CCR8 ligands CCL1 and CCL18 also have increased expression in several types of cancer.

To improve cancer treatment, there is a need for new actives and therapies aiming at selectively depleting tumor-infiltrating Tregs.

There is also a need for a new agent with improved cancer-specific Tregs depletion ability via antibody-dependent cellular cytotoxicity (ADCC) compared to simple anti-CCR8 antibodies, ensuring more effective targeting and elimination of regulatory T cells in the tumor microenvironment.

There is also a need for a new agent that acts as a Treg starver, effectively depriving regulatory T cells of necessary survival signals, thereby reducing their suppressive activity on the immune response against tumors.

There is also a need for a new agent with the ability to stabilize the CCR8 receptor at the membrane level of Tregs, preventing its internalization and ensuring sustained targeting by therapeutic agents.

There is moreover a need for a new agent that preferentially targets CCR8+ cells without affecting CD25+ cells, minimizing collateral damage to non-tumor-related Treg cells and reducing potential side effects.

There is also a need for a new agent with the ability to abolish the migration of Tregs towards tumors, effectively blocking their recruitment and accumulation in the tumor microenvironment, which contributes to immune evasion by cancer cells.

There is furthermore a need for a robust new antitumor agent which can directly or indirectly enhance the immune system's ability to recognize and destroy cancer cells.

There is a need for innovative strategies to overcome the limitations of current cancer immunotherapies by addressing specific challenges such as Tregs-mediated immunosuppression and improving the specificity and efficacy of therapeutic antitumoral interventions.

The present invention aims to address these challenges by providing new compounds, in the form of new immunocytokines.

### SUMMARY OF THE INVENTION

A first object of the present invention relates to a fusion protein comprising an anti-CCR8 antibody, or a fragment thereof, and at least one IL-2 variant which is a Treg-specific IL-2 receptor antagonist;
wherein the N-terminus of the IL-2 variant is fused to the C-terminus of the anti-CCR8 antibody heavy chain.

A fusion protein according to the invention may induce Treg apoptosis.

A fusion protein according to the invention may deplete selectively tumor-infiltrating Tregs and in particular tumor-associated Tregs.

The Treg-specific IL-2 receptor antagonist of a fusion protein according to the invention may comprise at least one, and in particular one, amino acid deletion at a position selected from S4, S5 or S6, the indicated positions being determined by alignment with wild-type human IL-2 set forth as sequence SEQ ID NO: 1.

The Treg-specific IL-2 receptor antagonist of a fusion protein according to the invention may comprise the substitutions K9E, L12E, H16R, L19R, M23L, N26K, S87N, V91K, E95K, N119K and T123A;
and further comprise:
(i) the substitutions S127K and Y31P; or
(ii) the substitutions K49Q, E52S, R81E, D84N, T131R and L132S; or
(iii) the substitution S127K;
the indicated positions being determined by alignment with wild-type human IL-2 set forth as sequence SEQ ID NO: 1.

The Treg-specific IL-2 receptor antagonist of a fusion protein according to the invention may comprise the substitutions K9E, L12E, H16R, L19R, M23L, N26K, S87N, V91K, E95K, N119K and T123A; and further comprise the substitutions S127K and Y31P.

The anti-CCR8 antibody of a fusion protein according to the invention may comprise:
(i) a heavy chain comprising:
   - a H-CDR1 having a sequence selected from the group consisting of the sequences set forth as SEQ ID NO: 3 and SEQ ID NO: 18;
   - a H-CDR2 having a sequence selected from the group consisting of the sequences set forth as SEQ ID NO: 4, SEQ ID NO: 11, SEQ ID NO: 19, SEQ ID NO: 26 and SEQ ID NO: 30; and
   - a H-CDR3 having a sequence selected from the group consisting of the sequences set forth as SEQ ID NO: 5, SEQ ID NO: 12, SEQ ID NO: 20, SEQ ID NO: 27, SEQ ID NO: 31 and SEQ ID NO: 34;
      and
(ii) a light chain comprising:
   - a L-CDR1 having a sequence selected from the group consisting of the sequences set forth as SEQ ID NO: 7, SEQ ID NO: 14, SEQ ID NO: 22 and SEQ ID NO: 36;
   - a L-CDR2 having a sequence selected from the group consisting of the sequences set forth as SEQ ID NO: 8, SEQ ID NO: 15 and SEQ ID NO: 23; and
   - a L-CDR3 having a sequence selected from the group consisting of the sequences set forth as SEQ ID NO: 9, SEQ ID NO: 16, SEQ ID NO: 24 and SEQ ID NO: 37;
or variants thereof having one or more conservative substitutions in one or more of these CDRs.

The anti-CCR8 antibody of a fusion protein according to the invention may comprise:
(i) a heavy chain comprising:
   - a H-CDR1 having the sequence set forth as SEQ ID NO: 3;
   - a H-CDR2 having a sequence selected from the group consisting of the sequences set forth as SEQ ID NO: 4 and SEQ ID NO: 11; and
   - a H-CDR3 having a sequence selected from the group consisting of the sequences set forth as SEQ ID NO: 5 and SEQ ID NO: 12;
      and
      a light chain comprising:
      - a L-CDR1 having a sequence selected from the group consisting of the sequences set forth as SEQ ID NO: 7 and SEQ ID NO: 14;
      - a L-CDR2 having a sequence selected from the group consisting of the sequences set forth as SEQ ID NO: 8 and SEQ ID NO: 15; and
      - a L-CDR3 having a sequence selected from the group consisting of the sequences set forth as SEQ ID NO: 9 and SEQ ID NO: 16;
         or
(ii) a heavy chain comprising:
   - a H-CDR1 having the sequence set forth as SEQ ID NO: 18 ;
   - a H-CDR2 having a sequence selected from the group consisting of the sequences set forth as SEQ ID NO: 19 and SEQ ID NO: 26; and
   - a H-CDR3 having a sequence selected from the group consisting of the sequences set forth as SEQ ID NO: 20 and SEQ ID NO: 27;
      and
      a light chain comprising:
      - a L-CDR1 having the sequence set forth as SEQ ID NO: 22;
      - a L-CDR2 having the sequence set forth as SEQ ID NO: 23; and
      - a L-CDR3 having the sequence set forth as SEQ ID NO: 24;
         or
(iii) a heavy chain comprising:
   - a H-CDR1 having the sequence set forth as SEQ ID NO: 3;
   - a H-CDR2 having the sequence set forth as SEQ ID NO: 30; and
   - a H-CDR3 having a sequence selected from the group consisting of the sequences set forth as SEQ ID NO: 31 and SEQ ID NO: 34;
      and
      a light chain comprising:
      - a L-CDR1 having a sequence selected from the group consisting of the sequences set forth as SEQ ID NO: 7 and SEQ ID NO: 36;
      - a L-CDR2 having a sequence selected from the group consisting of the sequences set forth as SEQ ID NO: 8 and SEQ ID NO: 15; and
      - a L-CDR3 having a sequence selected from the group consisting of the sequences set forth as SEQ ID NO: 9 and SEQ ID NO: 37;
or variants thereof having one or more conservative substitutions in one or more of these CDRs.

The anti-CCR8 antibody of a fusion protein according to the invention may comprise:
(i) a heavy chain comprising:
   - a H-CDR1 having the sequence set forth as SEQ ID NO: 3;
   - a H-CDR2 having a sequence selected from the group consisting of the sequences set forth as SEQ ID NO: 4 and SEQ ID NO: 11, and being in particular SEQ ID NO: 11; and
   - a H-CDR3 having a sequence selected from the group consisting of the sequences set forth as SEQ ID NO: 5 and SEQ ID NO: 12, and being in particular SEQ ID NO: 12;
      and
(ii) a light chain comprising:
   - a L-CDR1 having a sequence selected from the group consisting of the sequences set forth as SEQ ID NO: 7 and SEQ ID NO: 14, and being in particular SEQ ID NO: 14;
   - a L-CDR2 having a sequence selected from the group consisting of the sequences set forth as SEQ ID NO: 8 and SEQ ID NO: 15, and being in particular SEQ ID NO: 15; and
   - a L-CDR3 having a sequence selected from the group consisting of the sequences set forth as SEQ ID NO: 9 and SEQ ID NO: 16, and being in particular SEQ ID NO: 16;
or variants thereof having one or more conservative substitutions in one or more of these sequences.

A fusion protein according to the invention may be such that:
(i) the variable domain of the heavy chain of the anti-CCR8 antibody, or fragment thereof, comprises, and in particular consists in, an amino acid sequence selected from the group consisting of the sequences set forth as SEQ ID NO: 2, SEQ ID NO: 10, SEQ ID NO: 17, SEQ ID NO: 25, SEQ ID NO: 29 and SEQ ID NO: 33;
   and
(ii) the variable domain of the light chain of the anti-CCR8 antibody, or fragment thereof, comprises, and in particular consists in, an amino acid sequence selected from the group consisting of the sequences set forth as SEQ ID NO: 6, SEQ ID NO: 13, SEQ ID NO: 21, SEQ ID NO: 28, SEQ ID NO: 32 and SEQ ID NO: 35;
or variants thereof having one or more conservative substitutions in one or more of these sequences.

In particular, a fusion protein according to the invention may be such that:
(i) the variable domain of the heavy chain of the anti-CCR8 antibody, or fragment thereof, comprises, and in particular consists in, an amino acid sequence selected from the group consisting of the sequences set forth as SEQ ID NO: 2 and SEQ ID NO: 10, and is more particularly SEQ ID NO: 10;
   and
(ii) the variable domain of the light chain of the anti-CCR8 antibody, or fragment thereof, comprises, and in particular consists in, an amino acid sequence selected from the group consisting of the sequences set forth as SEQ ID NO: 6 and SEQ ID NO: 13, and is more particularly SEQ ID NO: 13,
or variants thereof having one or more conservative substitutions in one or more of these sequences.

The anti-CCR8 antibody fragment of a fusion protein according to the invention may be selected from the group consisting of a Fv, Fab, F(ab')2, Fab', dsFv, scFv, sc(Fv)2 and a diabody.

The anti-CCR8 antibody of a fusion protein according to the invention may be devoid of fucosyl residues.

The IL-2 variant of a fusion protein according to the invention may have at least 85% sequence identity, in particular at least 90% sequence identity, more particularly at least 95% sequence identity, with any one of SEQ ID NO: 38, SEQ ID NO: 39 and SEQ ID NO: 40.

A fusion protein according to the invention may further comprise a linker between the antibody heavy chain C-terminus and the IL-2 variant N-terminus, preferably a linker comprising, in particular consisting in, the sequence set forth as SEQ ID NO: 41.

Another object of the invention is a pharmaceutical composition comprising, in a pharmaceutically acceptable medium, at least a fusion protein according to the invention.

A further object of the invention is a fusion protein according to the invention, or a pharmaceutical composition according to the invention, for use in the treatment of cancer.

The cancer may be selected from the group consisting of Melanoma; renal cancer, in particular Renal Cell Carcinoma; Colorectal Cancer; liver cancer, in particular Hepatocellular Carcinoma; lung cancer, in particular Non-Small Cell Lung Cancer; mesothelioma, in particular Malignant Pleural Mesothelioma; Esophageal Cancer, in particular esophageal squamous cell carcinoma or adenocarcinoma; Head and Neck cancer, in particular Head and Neck Squamous Cell Carcinoma; urothelial cancer, in particular urothelial carcinoma; Lymphoma, in particular Primary Hodgkin Lymphoma or Large B Cell Lymphoma; Gastric Cancer, in particular gastric or gastroesophageal junction adenocarcinoma; Cervical Cancer; Merkel Cell Carcinoma; Endometrial cancer, in particular endometrial Carcinoma; skin cancer, in particular cutaneous squamous cell carcinoma; Breast cancer, in particular Triple Negative Breast Cancer or Breast invasive Carcinoma; Pancreatic cancer, in particular pancreatic Adenocarcinoma; Thymoma; Prostate cancer, in particular prostate adenocarcinoma; Ovarian cancer, in particular ovarian serous cystadenocarcinoma; Thyroid cancer, in particular thyroid carcinoma, and Sarcoma.

A fusion protein for use or a pharmaceutical composition for use according to the invention may be implemented in combination with at least one immune check-point inhibitor; in particular at least one selected from the group consisting of anti-PD-1, anti-PDL-1 or anti-CTLA-4 monoclonal antibodies.

### BRIEF DESCRIPTION OF THE FIGURES

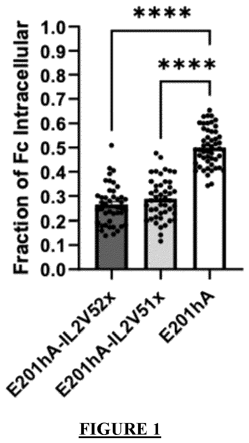

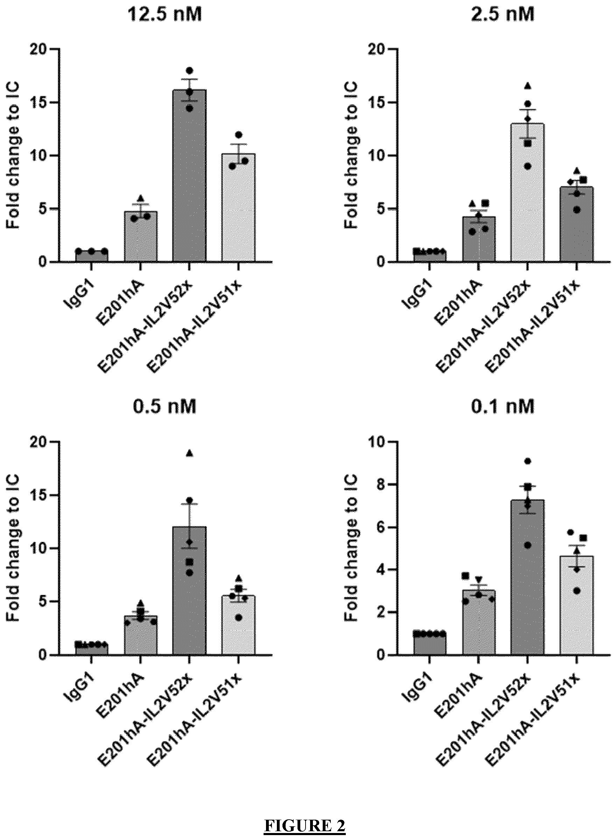

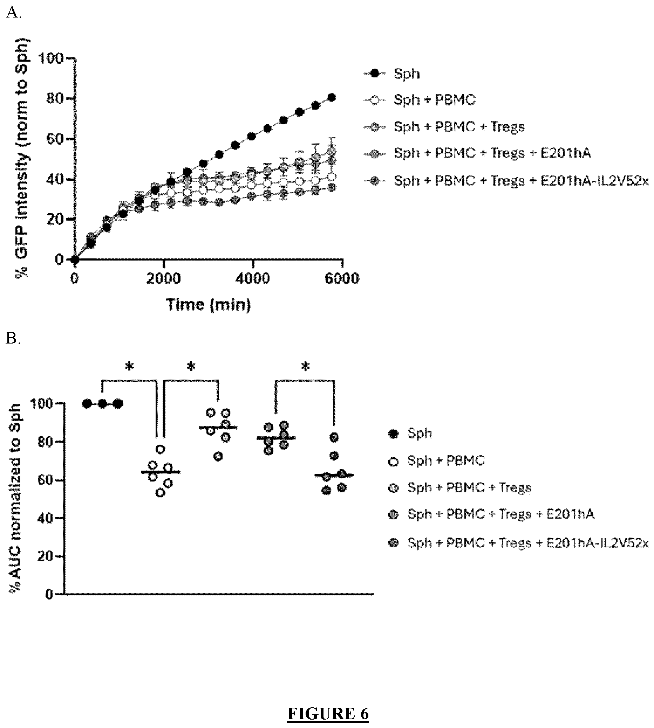

**Figure 1** shows the fraction of an intracellular molecule as detected by a fluorescently labeled anti-human Fc antibody (ordinate) in expanded Tregs expressing CCR8 on their surface and incubated with the following compounds, from left to right on the abscissa:
   - a fusion protein according to the invention (comprising two cytokines, i.e. bivalent): E201hA-II,2V52x (i.e. humanized (h) and afucosylated (A) antibody E201 fused to 2 (2x) IL-2V5 muteins (IL2V5));
   - a fusion protein according to the invention (comprising one cytokine, i.e. monovalent): E201hA-IL2V51x (i.e. humanized (h) and afucosylated (A) antibody E201 fused to 1 (1x) IL-2V5 mutein (IL2V5));
   - an afucosylated anti-CCR8 antibody (E201hA) (i.e. humanized (h) and afucosylated (A) antibody E201) as defined herein but not fused to a cytokine (outside of the invention).
   Kruscal-Wallis test, **** corresponds to p-value < 0.0001
**Figure 2** shows the increased ADCC activity of bi and monovalent fusion proteins (immunocytokines) of the invention compared to standard afucosylated monoclonal antibody tested at various concentrations (0.1 nM, 0.5 nM, 2.5 nM or 12.5 nM) in a reporter assay.
   Ordinate: Fold change as normalized to IgG1 isotype control.
   Abscissa: from left to right: isotype control (control): IgG1; standard antibody (comparative): E201hA (i.e. humanized (h) and afucosylated (A) antibody E201); immunocytokine according to the invention (comprising two cytokines, i.e. bivalent): E201hA-II,2V52x; immunocytokine according to the invention (comprising one cytokine, i.e. monovalent): E201hA-IL2V51x.

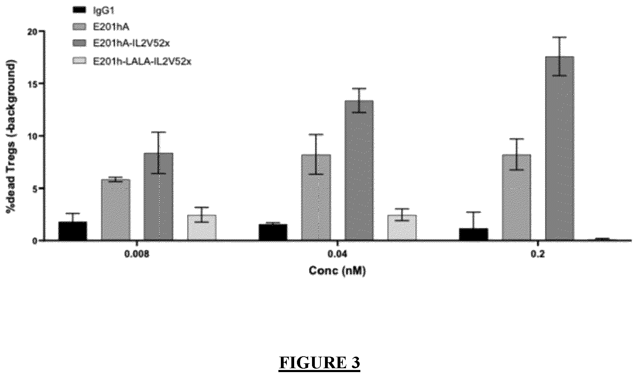

**Figure 3** shows the immunocytokines according to the invention demonstrate higher ADCC activity than standard antibodies in NK-based ADCC assay.
   Ordinate: % of dead Tregs (- background)
   Abscissa: tested concentrations (from left to right) : 0.008nM, 0.04 nM or 0.2 nM of (from left to right for each concentration) isotype control (control): IgG1; standard antibody (comparative): E201hA; immunocytokine according to the invention (comprising two cytokines, i.e. bivalent): E201hA-II,2V52x; negative control immunocytokine devoid of ADCC activity (comprising two cytokines and the E201h antibody in an Fc-silenced IgG1 backbone carrying the L234A and L235A mutations): E201h-LALA-IL2V52x, comparative.

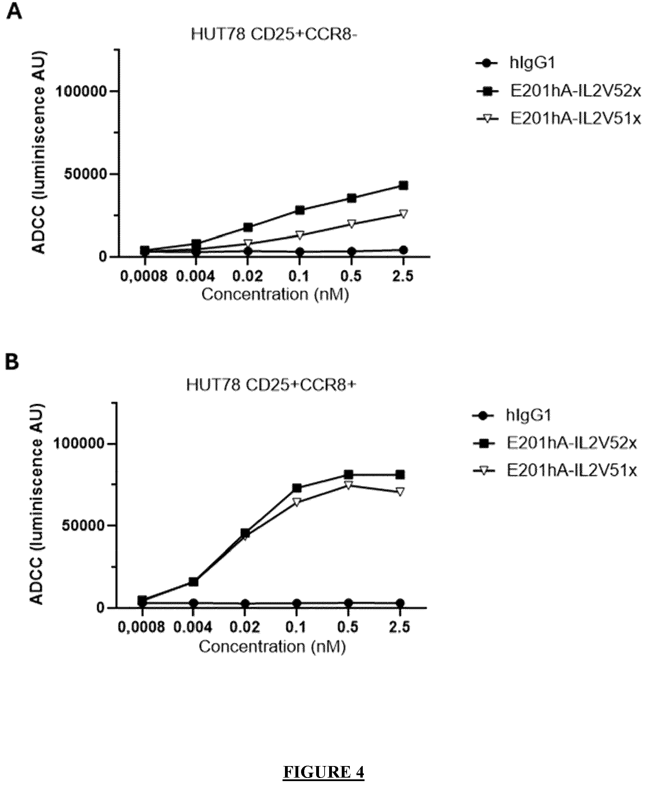

**Figure 4** shows the specificity of immunocytokines of the invention for CD25+CCR8+ cells but not for CD25+CCR8- cells. Figure 4A represents the ADCC results obtained on HUT78 CD25+ CCR8- cells, Figure 4B represents the ADCC results obtained on HUT78 CD25+ CCR8+ cells with the following molecules: human isotype control (control): hIgG1; standard antibody (comparative): E201hA; immunocytokine according to the invention (comprising one cytokine, i.e. monovalent): E201hA-IL2V51x; immunocytokine according to the invention (comprising two cytokines, i.e. bivalent): E201 hA-II,2V52x.
   Abscissa: concentration of tested molecule from 0.0008 nM to 2.5 nM (0.0008 nM, 0.004 nM, 0.02 nM, 0.1 nM, 0.5 nM and 2.5 nM).
   Ordinate: ADCC (luminescence Arbitrary Unit (AU)).

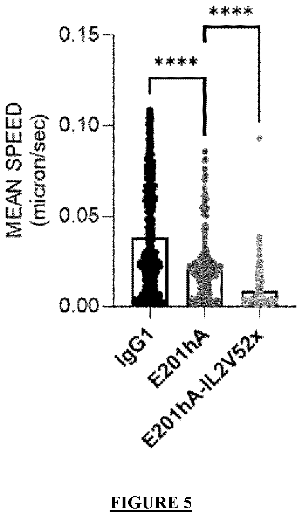

**Figure 5** shows the impact of various molecules (as indicated below) on Tregs migration.
   Ordinate: mean speed of the Tregs (micron/sec)
   Abscissa: from left to right: human isotype control (control): hIgG1; standard antibody (comparative): E201hA; a fusion protein according to the invention (comprising two cytokines, i.e. bivalent): E201hA-II,2V52x.
   Kruscal-Wallis test, **** corresponds to p-value < 0.0001
**Figure 6** shows the evaluation of the anti-tumor activity of various molecules in a model of spheroids formed by GFP expressing A549 human lung carcinoma cells (1000 cells at D0) and in the presence of 10000 effector cells (PBMC previously activated with anti-CD3) as well as expanded Tregs stimulated by anti-TNF-R2 antibody to express CCR8.
   Five situations were compared:
   - spheroids with no PBMC (Sph);
   - spheroids with PBMC (Sph + PBMC);
   - spheroids with PBMC and Tregs (Sph + PBMC + Tregs);
   - spheroids with PBMC and Tregs and with standard antibody (Sph + PBMC + Tregs + standard antibody E201hA);
   - spheroids with PBMC and Tregs and with a bivalent fusion protein according to the invention: E201hA-IL2V52x (Sph + PBMC + Tregs + E201hA-IL2V52x).
Figure 6A: Ordinate: % GFP intensity (norm to Sph). Abscissa: Time (min).
Figure 6B: Ordinate: % Area Under the Curve (AUC) normalized to Spheroids with no PBMC. Abscissa: from left to right in the order mentioned above.
   Wilcoxon test, * corresponds to p-value < 0.05
**Figure 7** shows the evaluation of the anti-tumor activity of various molecules in ex vivo human tumor slice cultures obtained from a breast tumor.
   Ordinate: % caspase activity in Epcam+ areas
   Abscissa: from left to right: human isotype control hIgG 1 (Ctrl); standard antibody E201hA; bivalent afucosylated immunocytokine according to the invention: E201hA-II,2V52x; negative control immunocytokine devoid of ADCC activity (comprising two cytokines and the E201h antibody in an Fc-silenced IgG1 backbone carrying the L234A and L235A mutations): E201h-LALA-IL2V52x, comparative.

### DETAILED DESCRIPTION OF THE INVENTION

The inventors have been able to generate new fusion proteins, also named immunocytokines, combining a plurality of superior, surprising and advantageous properties in the treatment of cancers.

Surprisingly, the inventors have indeed shown that the new immunocytokine are able to preferentially, and in particular selectively, deplete tumor-infiltrating Tregs. The new immunocytokines moreover have improved cancer-specific Tregs depletion ability via ADCC compared to simple anti-CCR8 antibodies, ensuring more effective targeting and elimination of regulatory T cells in the tumor microenvironment. The immunocytokines according to the invention are moreover able to act as Treg starvers, effectively depriving Tregs of necessary survival signals, thereby inducing their cell death.

The immunocytokines according to the invention moreover have the ability to stabilize the CCR8 receptor at the membrane level of Tregs, preventing its internalization and ensuring sustained targeting by therapeutic agents, in particular by the immunocytokines of the invention themselves. The immunocytokines according to the invention are moreover able to preferentially target CCR8+ and CCR8+CD25+ cells, minimizing collateral damage to non-tumor-related Tregs, which only express CD25, and reducing the potential side effects. They are furthermore able to abolish the migration of Tregs towards tumors, effectively blocking their recruitment and accumulation in the tumor microenvironment, which contributes to immune evasion by cancer cells. The immunocytokines of the invention have also shown robust antitumor activities in several experimental systems through their ability to directly or indirectly enhance the immune system's ability to recognize and destroy cancer cells and thus allowed overcoming the limitations of current cancer immunotherapies by addressing Tregs-mediated immunosuppression, which will allow improving the specificity and efficacy of therapeutic antitumoral interventions.

### Definitions

As used herein *"regulatory T cells"*, *"Treg"* or "*Tregs*" refer to CD3+CD4+Foxp3+ T cells, including CD3+CD4+Foxp3+CD25+ and CD3+CD4+Foxp3+CD25- cells.

As used herein, "*effector cells*" refer to effector T cells and NK cells. Effector T cells (Teff or Teffs) refer to one or more of conventional T (Tconv) cells and CD8+ T cells. Tconv are CD3+CD4+Foxp3- cells. CD8+ T cells are CD3+CD8+ cells. NK cells are CD3-CD16+ cells.

As used herein, "*Tumor-infiltrating Tregs*" are regulatory T cells (Tregs) that have migrated into a tumor. These cells play a role in suppressing the immune response against the tumor, allowing it to grow and evade the body's natural defense mechanisms. As used herein, "*tumor-associated Treg*" refers to activated Treg among tumor-infiltrating Tregs. In some particular embodiments, "tumor-associated Treg" refers to a distinct and isolated population (or group, subset or cluster) of CD4+ Foxp3+ cells that distinguishes from the heterogeneous pool of Tregs in that: (i) it is increased in the tumor, and eventually also in the tumor draining-lymph node(s); (ii) it is enriched with clonally expanded TCR specificities in the diseased-tissue; and (iii) it is enriched with a transcriptomic signature of T cell Receptor (TCR) triggering, cell activation and expansion, as disclosed in WO2021/165546.

According to the present invention, "*antibody*" or "*immunoglobulin*" have the same meaning, and will be used equally in the present invention. The term "*antibody*" as used herein refers to immunoglobulin molecules and immunologically active portions of immunoglobulin molecules, i.e., molecules that contain an antigen binding site that immunochemically binds an antigen. As such, the term antibody encompasses not only whole antibody molecules, but also variants (including derivatives) of antibodies. In natural antibodies, two heavy chains are linked to each other by disulfide bonds and each heavy chain is linked to a light chain by a disulfide bond. There are two types of light chain, lambda (l) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs. The well-known IMGT numbering system is used in the present text for defining antibodies according to the invention, and in particular to define their CDRs.

In the present text, the term "*antibody*" is used in the broadest sense and includes fully assembled antibodies, monoclonal antibodies, multi-specific antibodies (e.g., bispecific antibodies), antibody fragments that can bind CCR8, and recombinant peptides comprising the forgoing as long as they exhibit the desired biological activity defined herein.

The term "*chimeric antibody*" refers to an antibody which comprises a VH domain, and preferably a VL domain, of an antibody which are derived from one species and the constant domain which is derived from another species, for example an antibody in which the variable region sequences are derived from a mouse antibody and the constant region sequences are derived from a human antibody.

According to the invention, the term "*humanized antibody*" refers to an antibody having variable region framework and constant regions from a human antibody but which retains the mouse sequence origin of the CDRs of the variable V region(s).

The terms "*monoclonal antibody*" or "*monoclonal antibody composition*" as used herein refer to a preparation of antibody molecules of single molecular composition. A monoclonal antibody composition displays a single binding specificity and affinity for a particular epitope.

The terms "*anti-CCR8 antibody*" refer to an antibody directed against CCR8, and in particular against human CCR8.

By the term "*specifically binds to"* or "*is specific for*" as used herein with respect to an antibody, is meant an antibody or antibody fragment, which recognizes and binds to a specific antigen, but does not recognize or bind in significant amount (detectably bind) to other molecules present in the sample or to other molecules to which the antibody may come into contact in an organism, while detectably binding to its specific antigen. The specific binding of an antibody to its antigen may be determined by standard assays such as immunoassay. An antibody specifically binds to its antigen when it has a dissociation constant (KD) of 1 µM or less for its antigen in a standard KD determination assay. KD values are expressed as molar concentration (M); KD for antibodies are usually determined by Surface plasmon resonance using Biacore assay.

A "*fragment of an antibody*" herein refers to a fragment of an intact antibody that retains the ability to specifically binds to a given antigen/ligand. Examples of fragment of an antibody include a Fab fragment, a monovalent fragment consisting of the VL, VH, CL and CH1 domains; a Fab' fragment, a monovalent fragment consisting of the VL, VH, CL, CH1 domains and hinge region; a F(ab')2 fragment, a bivalent fragment comprising two Fab' fragments linked by a disulfide bridge at the hinge region; an Fd fragment consisting of VH-CH1 domains of a single arm of an antibody, in particular of a heavy chain of an antibody; a single domain antibody (sdAb) fragment (Ward et al., 1989 Nature 341:544-546), which consists of a VH domain or a VL domain; and an isolated complementary determining region (CDR). Furthermore, although the two domains of the Fv fragment, VL and VH, are coded for by separate genes, they can be joined, using recombinant methods, by an artificial peptide linker that enables them to be made as a single protein chain in which the VL and VH regions pair to form monovalent molecules (known as single chain Fv (scFv); see, e.g., Bird et al., 1989 Science 242:423-426; and Huston et al., 1988 proc. Natl. Acad. Sci. 85:5879-5883). "dsFv" is a VH::VL heterodimer stabilized by a disulfide bond.

Divalent and multivalent antibody fragments can form either spontaneously by association of monovalent scFvs or can be generated by coupling monovalent scFvs by a peptide linker, such as divalent sc(Fv)2. Such single chain antibodies include one or more antigen-binding portions or fragments of an antibody. These antibody fragments are obtained using conventional techniques known to those skilled in the art, and the fragments are screened for utility in the same manner as are intact antibodies.

A unibody is another type of antibody fragment lacking the hinge region of IgG4 antibodies. The deletion of the hinge region results in a molecule that is essentially half the size of traditional IgG4 antibodies and has a univalent binding region rather than the bivalent binding region of IgG4 antibodies. Further details on UniBodies may be obtained by reference to WO 2007/059782, which is incorporated by reference in its entirety.

Fragments of antibodies can be incorporated into single domain antibodies, SMIP, maxibodies, minibodies, intrabodies, diabodies, triabodies and tetrabodies (see, e.g., Hollinger and Hudson, 2005, Nature Biotechnology, 23, 9, 1126-1136). The term "diabodies" "tribodies" or "tetrabodies" refers to small antibody fragments with multivalent antigen-binding sites (2, 3 or four), which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Further details on domain antibodies and methods of their production are found in US 6,291,158; 6,582,915; 6,593,081; 6,172,197; and 6,696,245; US 2004/0110941; EP 1433846, 0368684 and 0616640; WO 2005/035572, 2004/101790, 2004/081026, 2004/058821, 2004/003019 and 2003/002609, each of which is herein incorporated by reference in its entirety.

Antigen binding fragments can be incorporated into single chain molecules comprising a pair of tandem Fd segments (VH-CH1-VH-CH1) which, together with complementary light chain polypeptides, form a pair of antigen binding regions (Zapata et al., 1995 Protein Eng. 8(10); 1057-1062 and U.S. Pat. No. 5,641,870).

Fab fragments can be obtained by treating an antibody with a protease, papain. Also, the Fab can be produced by inserting DNA encoding Fab of the antibody into a vector for prokaryotic expression system, or for eukaryotic expression system, and introducing the vector into a procaryote or eucaryote (as appropriate) to express the Fab.

F(ab')2 can be obtained by treating an antibody with a protease, pepsin. Also, the F(ab')2 can be produced by binding Fab' described below via a thioether bond or a disulphide bond.

Fab' can be obtained by treating F(ab')2 with a reducing agent, dithiothreitol. Also, the Fab' can be produced by inserting DNA encoding Fab' fragment of the antibody into an expression vector for prokaryote, or an expression vector for eukaryote, and introducing the vector into a prokaryote or eukaryote (as appropriate) to perform its expression.

An antigen-binding fragment may be variable heavy chain of a single domain antibody (VHH). Some VHHs may also be known as Nanobodies. Camelid single domain antibody (sdAb) is one of the smallest known antigen-binding antibody fragments (see, e.g., Hassanzadeh- Ghassabeh et al., Nanomedicine (Lond), 8:1013-26 (2013)). A basic VHH has the following structure from the N-terminus to the C-terminus: FR1-CDR1-FR2-CDR2-FR3-CDR3-FR4, in which FR1 to FR4 refer to framework regions 1 to 4, respectively, and in which CDR1 to CDR3 refer to the complementarity determining regions (CDR) 1 to 3.

Antibody fragments according to the invention may in particular be selected from the group consisting of Fv, Fab, F(ab')2, Fab', dsFv, scFv, sc(Fv)2 and diabodies.

They may be produced by recombinant DNA techniques or by enzymatic or chemical cleavage of intact antibodies, according to methods well known to the man skilled in the art.

As previously mentioned, an antibody according to the invention may be a multi-specific antibody, and in particular a bispecific antibody.

A "*multi-specific antibody*" refers to monoclonal antibody that has binding specificities for at least two different epitopes of one antigen or for at least two distinct antigens. In the present application, at least one of the antigens is CCR8 as mentioned above. In some embodiments, a multi-specific antibody may be a bispecific antibody; a trispecific antibody; a tetraspecific antibody; a pentaspecific antibody; or a hexaspecific antibody, in particular a bispecific antibody.

The expression "*bispecific antibody*" refers to a molecule which combines the antigen-binding sites of two antibodies within a single molecule. Thus, a bispecific antibody is able to bind two different antigens or to two distinct epitopes of a same antigen, simultaneously. The distinct epitope may be overlapping epitopes or non-overlapping epitopes.

The term "*treatment*" or "*therapy*" refers to administering an active agent with the purpose to cure, heal, alleviate, relieve, alter, remedy, ameliorate, improve, or affect a condition (e.g., a disease), the symptoms of the condition, or to prevent or delay the onset of the symptoms, complications, biochemical indicia of a disease, or otherwise arrest or inhibit further development of the disease, condition, or disorder in a statistically significant manner.

According to the invention, the terms "*patient*"*,* "*individual*"*,* "*patient in need thereof*" or "*individual in need thereof*" are equivalent and are intended for a human or non-human mammal affected or likely to be affected with the disease or disorder considered, in particular affected with cancer in an individual in need thereof. Said individual is in particular a mammal, preferably a human being.

The term interleukin-2 or IL-2, also known as TCGF or lymphokine, refers to a protein encoded by the IL-2 gene in a mammalian genome. IL-2 is expressed as a precursor containing a N-terminal signal peptide (20 amino acids) which is cleaved to yield the mature protein (IL-2). Representative examples of IL-2 include without limitation, human (Gene ID: 3558), rat (Gene ID: 116562), cat (Gene ID: 751114), and mouse (Gene ID 16183) forms. As used herein, IL-2, refers to wild-type IL-2. Human IL-2 precursor has the 153 amino acid sequence UniProtKB/Swiss-Prot: P60568.1. Mature IL-2 has the 133 amino acid sequence from positions 21 to 153 of the precursor and corresponds to SEQ ID NO: 1.

In the context of the present invention, an IL-2 variant, also termed IL-2 mutein, refers to a mutant IL-2 comprising one or more mutations, i.e. insertions, deletions and/or substitutions, and in particular substitutions, as compared to wild-type IL-2. In the present invention, IL-2 variants have a modified biological activity as compared to wild-type IL-2. IL-2 variant is preferably human IL-2 variant. WT IL-2 may in particular have sequence SEQ ID NO: 1.

The term Treg-specific IL-2 receptor antagonist or IL-2 variant with IL-2 receptor antagonist activity specific for Tregs refers to an IL-2 variant which is a dominant negative IL-2 molecule that does not induce IL-2-mediated STAT-5 phosphorylation in cells bearing the IL-2R and competes with wild-type IL-2 for signaling through the trimeric alpha, beta, gamma IL-2R. The IL-2 receptor antagonist activity specific for Tregs to the IL-2 variant according to the invention is illustrated in the examples of the present application.

A fusion protein according to the invention (also termed immunocytokine) is implemented according to the present invention in a therapeutically effective amount.

By a "*therapeutically effective amount*" of the fusion protein of the invention is meant a sufficient amount of fusion protein to treat said cancer, at a reasonable benefit/risk ratio applicable to any medical treatment. It will be understood, however, that the total daily usage of the fusion of the present invention will be decided by the attending physician within the scope of sound medical judgment. The specific therapeutically effective dose level for any particular patient will depend upon a variety of factors including the disorder being treated and the severity of the disorder; activity of the specific antibody employed; the specific composition employed, the age, body weight, general health, sex and diet of the patient; the time of administration, route of administration, and rate of excretion of the specific antibody employed; the duration of the treatment; drugs used in combination or coincidental with the specific fusion protein employed; and like factors well known in the medical arts. For example, it is well known within the skill of the art to start doses of the compound at levels lower than those required to achieve the desired therapeutic effect and to gradually increase the dosage until the desired effect is achieved.

For each or the amino acid sequences of interest, reference sequences are described herein. The present description also encompasses amino acid sequences (e.g. enzyme amino acid sequences), having specific percentages of amino acid identity with a reference amino acid sequence.

For obvious reasons, in all the present description, a specific nucleic acid sequence or a specific amino acid sequence which complies with, respectively, the considered nucleotide or amino acid identity, should further lead to obtaining a protein (or enzyme) which displays the desired biological activity.

As used herein, the "*percentage of identity*" between two nucleic acid sequences or between two amino acid sequences is determined by comparing both optimally aligned sequences through a comparison window.

The portion of the nucleotide or amino-acid sequence in the comparison window may thus include additions or deletions (for example "gaps") as compared to the reference sequence (which does not include these additions or these deletions) so as to obtain an optimal alignment between both sequences. An IL-2 variant according to the invention comprises preferably at least one amino acid deletion, more preferably at a position selected from S4, S5 or S6, the indicated positions being determined by alignment with SEQ ID NO: 1.

The terms "*sequence homology*" or "*sequence identity*" or "*homology*" or "*identity*" are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region. A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman and Wunsch, J. Mol. Biol., 48: 443-453 (1970)). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. Longden J. and Bleasby, A. Trends in Genetics 16, (6) pp276- 277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin and Altschul, Proc. Natl. Acad. Sci. USA, 90: 5873-5877 (1993)), with hmmalign (HMMER package, http://hmmer.org/) or with the CLUSTAL algorithm (Thompson et al., Nucleic Acids Res., 22: 4673-80 (1994)) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al., J. Mol. Biol., 215: 403-410 (1990). BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al., Nucleic Acids Res., 25: 3389-3402 (1997). When utilizing BLAST and Gapped BLAST programs, the default parameters of the respective programs are used. Sequence matching analysis may be supplemented by established homology mapping techniques like Shuffle-LAGAN (Brudno, Bioinformatics, 19 Suppl 1: 154-162 (2003)) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

A sequence "*at least 85*% *identical to a reference sequence*" is a sequence having, on its entire length, 85%, or more, in particular 86%, 87%, 88%, 89%, 90%, 91 %, 92%, 93%, 94%, 95%, 96%, 97%, 98% or 99% sequence identity with the entire length of the reference sequence.

In the present invention, amino acids are represented in the sequences on the basis of the commonly recognized and known single-letter code which is reminded hereafter:
Alanine: A; Arginine: R; Asparagine: N; Aspartic Acid: D; Cysteine: C; Glutamic Acid: E; Glutamine: Q; Glycine: G; Histidine: H; Isoleucine: I; Leucine: L; Lysine: K; Methionine: M; Phenylalanine: F; Proline: P; Serine: S; Threonine: T; Tryptophan: W; Tyrosine: Y and Valine: V.

Substitutions are designated herein by the one letter amino acid code followed by the substituting residue in one letter amino acid code. For example, K9E is a substitution of the lysine (K) residue at position 9 of a given sequence with a Glutamic acid (E) residue.

Substitutions may be conservative or non-conservative. Preferably, substitutions are conservative substitutions, in which one amino acid is substituted for another amino acid with similar structural and/or chemical properties.

"*Treating cancer*" includes, without limitation, reducing the number of cancer cells or the size of a tumor in the patient, reducing progression of a cancer to a more aggressive form (i.e. maintaining the cancer in a form that is susceptible to a therapeutic agent), reducing proliferation of cancer cells or reducing the speed of tumor growth, killing of cancer cells, reducing metastasis of cancer cells or reducing the likelihood of recurrence of a cancer in a subject. Treating a subject as used herein refers to any type of treatment that imparts a benefit to a subject afflicted with cancer or at risk of developing cancer or facing a cancer recurrence. Treatment includes improvement in the condition of the subject (e.g., in one or more symptoms), delay in the progression of the disease, delay in the onset of symptoms, slowing the progression of symptoms and others.

"a", "an", and "the" include plural referents, unless the context clearly indicates otherwise. As such, the term "a" (or "an"), "one or more" or "at least one" can be used interchangeably herein.

### Fusion proteins of the invention

The protein according to the invention which is a fusion of an anti-CCR8 antibody to a variant of IL-2 cytokine is an immunocytokine. A Treg specific IL-2 receptor antagonist is named herein IL-2 antagonist or IL-2 variant.

Indeed, as indicated above, fusion proteins of the invention are defined as comprising an anti-CCR8 antibody, or a fragment thereof, and at least one IL-2 variant, in particular one or two, the IL-2 variant being a Treg-specific IL-2 receptor antagonist.

In a fusion protein of the invention, the N-terminus of the IL-2 variant(s) is fused to the C-terminus of the antibody heavy chain.

A fusion protein according to the invention preferably induces Treg apoptosis.

A fusion protein according to the invention preferably depletes selectively tumor-infiltrating Tregs and in particular tumor-associated Tregs.

The immunocytokine according to the invention may use known anti-CCR8 antibodies, in particular therapeutic anti-CCR8 antibodies.

Preferably, new anti-CCR8 antibodies may be implemented, and in particular anti-CCR8 antibodies as defined below.

The antibody or fragment thereof as defined in the invention binds specifically to the CCR8 receptor that is preferentially expressed on tumor-infiltrating Tregs and in particular tumor-associated Tregs. In some embodiments, the antibody may further inhibit signaling through the receptor, which means that the antibody is a blocking, inhibitory or antagonist antibody.

Anti-CCR8 antibodies of fusion protein of the invention may be produced by any technique known in the art, such as, without limitation, any chemical, biological, genetic or enzymatic technique, either alone or in combination.

Knowing the amino acid sequence of the desired sequence, one skilled in the art can readily produce said antibodies, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions. Alternatively, antibodies of the invention can be synthesized by recombinant DNA techniques well-known in the art. For example, antibodies can be obtained as DNA expression products after incorporation of DNA sequences encoding the antibodies into expression vectors and introduction of such vectors into suitable eukaryotic or prokaryotic hosts that will express the desired antibodies, from which they can be later isolated using well-known techniques.

An antibody of a fusion protein of the invention comprises a heavy chain, and preferably a light chain. The heavy chain comprises a heavy chain variable region (VH) and the light chain, when present, comprises a light chain variable region (VL), preferably human or humanized VH and VL.

In some particular embodiments, the antibody further comprises an Ig constant region, including native sequence Ig constant region and variant Ig constant region. The Ig constant region is preferably human. The Ig constant region comprises at least one Ig constant domain; in particular at least CH1 and CL, more particularly CL and CH1 and further Fc domain (CH2 and/or CH3 domains). The Fc region may be from any Ig class or isotype, preferably human IgG isotype. The Fc region may be native sequence Fc region or variant Fc region. Variant human Fc constant region may comprise the M428L and N434S substitutions (LS) to enhance antibody half-life. The numbering of residues in the Fc region is that of the EU index of Kabat.

In some particular embodiments, the antibody comprises a human IgG1 Fc domain and/or a human Ig kappa light chain constant domain.

In some particular embodiments, the antibody is a full-length human or humanized antibody. The antibody is preferably an IgG, particularly IgG1. The corresponding immunocytokine has the classical Ig structure comprising two Ig heavy chains and two Ig light chains and a copy of IL-2 variant fused to each heavy chain C-terminal end.

The anti-CCR8 antibody according to the invention may in particular be defined as comprising:
(i) a heavy chain comprising:
   - a H-CDR1 having a sequence selected from the group consisting of the sequences set forth as SEQ ID NO: 3 and SEQ ID NO: 18 ;
   - a H-CDR2 having a sequence selected from the group consisting of the sequences set forth as SEQ ID NO: 4, SEQ ID NO: 11, SEQ ID NO: 19, SEQ ID NO: 26 and SEQ ID NO: 30; and
   - a H-CDR3 having a sequence selected from the group consisting of the sequences set forth as SEQ ID NO: 5, SEQ ID NO: 12, SEQ ID NO: 20, SEQ ID NO: 27, SEQ ID NO: 31 and SEQ ID NO: 34;
      and
(ii) a light chain comprising:
   - a L-CDR1 having a sequence selected from the group consisting of the sequences set forth as SEQ ID NO: 7, SEQ ID NO: 14, SEQ ID NO: 22 and SEQ ID NO: 36;
   - a L-CDR2 having a sequence selected from the group consisting of the sequences set forth as SEQ ID NO: 8, SEQ ID NO: 15 and SEQ ID NO: 23; and
   - a L-CDR3 having a sequence selected from the group consisting of the sequences set forth as SEQ ID NO: 9, SEQ ID NO: 16, SEQ ID NO: 24 and SEQ ID NO: 37;
or variants thereof having one or more conservative substitutions in one or more of these CDRs.

The antibody may more particularly comprise:
(i) a heavy chain comprising:
   - a H-CDR1 having the sequence set forth as SEQ ID NO: 3;
   - a H-CDR2 having a sequence selected from the group consisting of the sequences set forth as SEQ ID NO: 4 and SEQ ID NO: 11; and
   - a H-CDR3 having a sequence selected from the group consisting of the sequences set forth as SEQ ID NO: 5 and SEQ ID NO: 12;
      and
(ii) a light chain comprising:
   - a L-CDR1 having a sequence selected from the group consisting of the sequences set forth as SEQ ID NO: 7 and SEQ ID NO: 14;
   - a L-CDR2 having a sequence selected from the group consisting of the sequences set forth as SEQ ID NO: 8 and SEQ ID NO: 15; and
   - a L-CDR3 having a sequence selected from the group consisting of the sequences set forth as SEQ ID NO: 9 and SEQ ID NO: 16;
or variants thereof having one or more conservative substitutions in one or more of these CDRs.

The antibody may more particularly comprise:
(i) a heavy chain comprising:
   - a H-CDR1 having the sequence set forth as SEQ ID NO: 3;
   - a H-CDR2 having the sequence set forth as SEQ ID NO: 4; and
   - a H-CDR3 having the sequence set forth as SEQ ID NO: 5;
      and
(ii) a light chain comprising:
   - a L-CDR1 having the sequence set forth as SEQ ID NO: 7;
   - a L-CDR2 having the sequence set forth as SEQ ID NO: 8; and
   - a L-CDR3 having the sequence set forth as SEQ ID NO: 9; (corresponding to the sequences of antibody E201)
or variants thereof having one or more conservative substitutions in one or more of these CDRs.

The antibody may more particularly comprise:
(i) a heavy chain comprising:
   - a H-CDR1 having the sequence set forth as SEQ ID NO: 3;
   - a H-CDR2 having the sequence set forth as SEQ ID NO: 11; and
   - a H-CDR3 having the sequence set forth as SEQ ID NO: 12;
      and
(ii) a light chain comprising:
   - a L-CDR1 having the sequence set forth as SEQ ID NO: 14;
   - a L-CDR2 having the sequence set forth as SEQ ID NO: 15; and
   - a L-CDR3 having the sequence set forth as SEQ ID NO: 16; (corresponding to the sequences of humanized antibody E201, i.e. E201h)
or variants thereof having one or more conservative substitutions in one or more of these CDRs.

The antibody may more particularly comprise:
(i) a heavy chain comprising:
   - a H-CDR1 having the sequence set forth as SEQ ID NO: 18;
   - a H-CDR2 having a sequence selected from the group consisting of the sequences set forth as SEQ ID NO: 19 and SEQ ID NO: 26; and
   - a H-CDR3 having a sequence selected from the group consisting of the sequences set forth as SEQ ID NO: 20 and SEQ ID NO: 27;
      and
(ii) a light chain comprising:
   - a L-CDR1 having the sequence set forth as SEQ ID NO: 22;
   - a L-CDR2 having the sequence set forth as SEQ ID NO: 23; and
   - a L-CDR3 having the sequence set forth as SEQ ID NO: 24;
or variants thereof having one or more conservative substitutions in one or more of these CDRs.

The antibody may more particularly comprise:
(i) a heavy chain comprising:
   - a H-CDR1 having the sequence set forth as SEQ ID NO: 18;
   - a H-CDR2 having the sequence set forth as SEQ ID NO: 19; and
   - a H-CDR3 h having the sequence set forth as SEQ ID NO: 20; and
(ii) a light chain comprising:
   - a L-CDR1 having the sequence set forth as SEQ ID NO: 22;
   - a L-CDR2 having the sequence set forth as SEQ ID NO: 23; and
   - a L-CDR3 having the sequence set forth as SEQ ID NO: 24; (corresponding to the sequences of antibody E202)
or variants thereof having one or more conservative substitutions in one or more of these CDRs.

The antibody may more particularly comprise:
(i) a heavy chain comprising:
   - a H-CDR1 having the sequence set forth as SEQ ID NO: 18;
   - a H-CDR2 having the sequence set forth as SEQ ID NO: 26; and
   - a H-CDR3 h having the sequence set forth as SEQ ID NO: 27; and
(ii) a light chain comprising:
   - a L-CDR1 having the sequence set forth as SEQ ID NO: 22;
   - a L-CDR2 having the sequence set forth as SEQ ID NO: 23; and
   - a L-CDR3 having the sequence set forth as SEQ ID NO: 24; (corresponding to the sequences of humanized antibody E202)
or variants thereof having one or more conservative substitutions in one or more of these CDRs.

The antibody may more particularly comprise:
(i) a heavy chain comprising:
   - a H-CDR1 having the sequence set forth as SEQ ID NO: 3;
   - a H-CDR2 having the sequence set forth as SEQ ID NO: 30; and
   - a H-CDR3 having a sequence selected from the group consisting of the sequences set forth as SEQ ID NO: 31 and SEQ ID NO: 34;
      and
(ii) a light chain comprising:
   - a L-CDR1 having a sequence selected from the group consisting of the sequences set forth as SEQ ID NO: 7 and SEQ ID NO: 36;
   - a L-CDR2 having a sequence selected from the group consisting of the sequences set forth as SEQ ID NO: 8 and SEQ ID NO: 15; and
   - a L-CDR3 having a sequence selected from the group consisting of the sequences set forth as SEQ ID NO: 9 and SEQ ID NO: 37;
or variants thereof having one or more conservative substitutions in one or more of these CDRs.

The antibody may more particularly comprise:
(i) a heavy chain comprising:
   - a H-CDR1 having the sequence set forth as SEQ ID NO: 3;
   - a H-CDR2 having the sequence set forth as SEQ ID NO: 30; and
   - a H-CDR3 h having the sequence set forth as SEQ ID NO: 31;
      and
(ii) a light chain comprising:
   - a L-CDR1 having the sequence set forth as SEQ ID NO: 7;
   - a L-CDR2 having the sequence set forth as SEQ ID NO: 8; and
   - a L-CDR3 having the sequence set forth as SEQ ID NO: 9; (corresponding to the sequences of antibody E203)
or variants thereof having one or more conservative substitutions in one or more of these CDRs.

The antibody may more particularly comprise:
(i) a heavy chain comprising:
   - a H-CDR1 having the sequence set forth as SEQ ID NO: 3;
   - a H-CDR2 having the sequence set forth as SEQ ID NO: 30; and
   - a H-CDR3 h having the sequence set forth as SEQ ID NO: 34;
      and
(ii) a light chain comprising:
   - a L-CDR1 having the sequence set forth as SEQ ID NO: 36;
   - a L-CDR2 having the sequence set forth as SEQ ID NO: 15; and
   - a L-CDR3 having the sequence set forth as SEQ ID NO: 37; (corresponding to the sequences of humanized antibody E203)
or variants thereof having one or more conservative substitutions in one or more of these CDRs.

An anti-CCR8 antibody of the invention may more particularly be such that:
(i) the variable domain of the heavy chain of the anti-CCR8 antibody, or fragment thereof, comprises, and in particular consists in, an amino acid sequence selected from the group consisting of the sequences set forth as SEQ ID NO: 2, SEQ ID NO: 10, SEQ ID NO: 17, SEQ ID NO: 25, SEQ ID NO: 29 and SEQ ID NO: 33;
   and
(ii) the variable domain of the light chain of the anti-CCR8 antibody, or fragment thereof, comprises, and in particular consists in, an amino acid sequence selected from the group consisting of the sequences set forth as SEQ ID NO: 6, SEQ ID NO: 13, SEQ ID NO: 21, SEQ ID NO: 28, SEQ ID NO: 32 and SEQ ID NO: 35.

An anti-CCR8 antibody of the invention may more particularly be selected from the group consisting of:
(i) an antibody, or fragment thereof, in which the variable domain of the heavy chain comprises, and in particular consists in, the amino acid sequence set forth as SEQ ID NO: 2 and the variable domain of the light chain comprises, and in particular consists in, the amino acid sequence set forth as SEQ ID NO: 6 (antibody E201);
(ii) an antibody, or fragment thereof, in which the variable domain of the heavy chain comprises, and in particular consists in, the amino acid sequence set forth as SEQ ID NO: 10 and the variable domain of the light chain comprises, and in particular consists in, the amino acid sequence set forth as SEQ ID NO: 13 (humanized antibody E201, ie E201h);
(iii) an antibody, or fragment thereof, in which the variable domain of the heavy chain comprises, and in particular consists in, the amino acid sequence set forth as SEQ ID NO: 17 and the variable domain of the light chain comprises, and in particular consists in, the amino acid sequence set forth as SEQ ID NO: 21 (antibody E202);
(iv) an antibody, or fragment thereof, in which the variable domain of the heavy chain comprises, and in particular consists in, the amino acid sequence set forth as SEQ ID NO: 25 and the variable domain of the light chain comprises, and in particular consists in, the amino acid sequence set forth as SEQ ID NO: 28 (humanized antibody E202);
(v) an antibody, or fragment thereof, in which the variable domain of the heavy chain comprises, and in particular consists in, the amino acid sequence set forth as SEQ ID NO: 29 and the variable domain of the light chain comprises, and in particular consists in, the amino acid sequence set forth as SEQ ID NO: 32 (antibody E203); and
(vi) an antibody, or fragment thereof, in which the variable domain of the heavy chain comprises, and in particular consists in, the amino acid sequence set forth as SEQ ID NO: 33 and the variable domain of the light chain comprises, and in particular consists in, the amino acid sequence set forth as SEQ ID NO: 35 (humanized antibody E203).

In particular, an anti-CCR8 antibody of the invention may more particularly be such that:
(i) the variable domain of the heavy chain of the anti-CCR8 antibody, or fragment thereof, comprises, and in particular consists in, an amino acid sequence selected from the group consisting of the sequences set forth as SEQ ID NO: 2 and SEQ ID NO: 10, and is more particularly SEQ ID NO: 10;
   and
(ii) the variable domain of the light chain of the anti-CCR8 antibody, or fragment thereof, comprises, and in particular consists in, an amino acid sequence selected from the group consisting of the sequences set forth as SEQ ID NO: 6 and SEQ ID NO: 13, and is more particularly SEQ ID NO: 13.

As mentioned above, an antibody of the invention comprises variants having one or more conservative substitutions in one or more of the indicated CDRs and/or VH and/or VL regions.

In particular, it is contemplated that antibodies or antigen-binding fragments thereof may have 1, 2, 3, 4, 5, 6, or more alterations in the amino acid sequence of 1, 2, 3, 4, 5, or 6 CDRs of antibodies provided herein, in particular in one or more of anyone of the CDR of sequences SEQ ID NO: 3-5, 7-9, 11, 12, 14-16, 18-20, 22-24, 26, 27, 30, 31, 34, 36 and 37 and/or in anyone of the VH and/or VL of sequences SEQ ID NO: 2, 6, 10, 13, 17, 21, 25, 28, 29, 32, 33 and 35.

In some particular embodiments, the anti-CCR8 antibody comprises a VH domain having at least 85 % sequence identity with a sequence selected from the group consisting of SEQ ID NO: 2, SEQ ID NO: 10, SEQ ID NO: 17, SEQ ID NO: 25, SEQ ID NO: 29 and SEQ ID NO: 33 and a VL domain having at least 85 % identity with a sequence selected from the group consisting of SEQ ID NO: 6, SEQ ID NO: 13, SEQ ID NO: 21, SEQ ID NO: 28, SEQ ID NO: 32 and SEQ ID NO: 35.

In particular, said VH and/or VH domain may have at least 86%, 87%, 88%, 89%, 90%, 91%, 92%, 93%, 94%, 95%, 96%, 97%, 98% or 99% sequence identity with said sequences, even more preferably at least 95%, 96%, 97%, 98% or 99% 98% or 99% sequence identity with said sequences.

Preferably, said VH and/or VH domain may have at least 86%, 87%, 88%, 89%, 90%, 91%, 92%, 93%, 94%, 95%, 96%, 97%, 98% or 99% sequence identity with said sequences and 100% identity with the corresponding 6 CDRs, i.e.:
(i) H-CDR1 having 100% sequence identity with sequence SEQ ID NO: 3, H-CDR2 having 100% sequence identity with sequence SEQ ID NO: 4 and H-CDR3 having 100% sequence identity with sequence SEQ ID NO: 5 in a VH having at least 85 % identity with the sequence set forth as SEQ ID NO: 2, and L-CDR1 having 100% sequence identity with sequence SEQ ID NO: 7, L-CDR2 having 100% sequence identity with sequence SEQ ID NO: 8 and L-CDR3 having 100% sequence identity with sequence SEQ ID NO: 9 in a VL having at least 85 % identity with the sequence set forth as SEQ ID NO: 6;
(ii) H-CDR1 having 100% sequence identity with sequence SEQ ID NO: 3, H-CDR2 having 100% sequence identity with sequence SEQ ID NO: 11 and H-CDR3 having 100% sequence identity with sequence SEQ ID NO: 12 in a VH having at least 85 % identity with the sequence set forth as SEQ ID NO: 10, and L-CDR1 having 100% sequence identity with sequence SEQ ID NO: 14, L-CDR2 having 100% sequence identity with sequence SEQ ID NO: 15 and L-CDR3 having 100% sequence identity with sequence SEQ ID NO: 16 in a VL having at least 85 % identity with the sequence set forth as SEQ ID NO: 13;
(iii) H-CDR1 having 100% sequence identity with sequence SEQ ID NO: 18, H-CDR2 having 100% sequence identity with sequence SEQ ID NO: 19 and H-CDR3 having 100% sequence identity with sequence SEQ ID NO: 20 in a VH having at least 85 % identity with the sequence set forth as SEQ ID NO: 17, and L-CDR1 having 100% sequence identity with sequence SEQ ID NO: 22, L-CDR2 having 100% sequence identity with sequence SEQ ID NO: 23 and L-CDR3 having 100% sequence identity with sequence SEQ ID NO: 24 in a VL having at least 85 % identity with the sequence set forth as SEQ ID NO: 21;
(iv) H-CDR1 having 100% sequence identity with sequence SEQ ID NO: 18, H-CDR2 having 100% sequence identity with sequence SEQ ID NO: 26 and H-CDR3 having 100% sequence identity with sequence SEQ ID NO: 27 in a VH having at least 85 % identity with the sequence set forth as SEQ ID NO: 25, and L-CDR1 having 100% sequence identity with sequence SEQ ID NO: 22, L-CDR2 having 100% sequence identity with sequence SEQ ID NO: 23 and L-CDR3 having 100% sequence identity with sequence SEQ ID NO: 24 in a VL having at least 85 % identity with the sequence set forth as SEQ ID NO: 28;
(v) H-CDR1 having 100% sequence identity with sequence SEQ ID NO: 3, H-CDR2 having 100% sequence identity with sequence SEQ ID NO: 30 and H-CDR3 having 100% sequence identity with sequence SEQ ID NO: 31 in a VH having at least 85 % identity with the sequence set forth as SEQ ID NO: 29, and L-CDR1 having 100% sequence identity with sequence SEQ ID NO: 7, L-CDR2 having 100% sequence identity with sequence SEQ ID NO: 8 and L-CDR3 having 100% sequence identity with sequence SEQ ID NO: 9 in a VL having at least 85 % identity with the sequence set forth as SEQ ID NO: 32; and
(vi) H-CDR1 having 100% sequence identity with sequence SEQ ID NO: 3, H-CDR2 having 100% sequence identity with sequence SEQ ID NO: 30 and H-CDR3 having 100% sequence identity with sequence SEQ ID NO: 34 in a VH having at least 85 % identity with the sequence set forth as SEQ ID NO: 33, and L-CDR1 having 100% sequence identity with sequence SEQ ID NO: 36, L-CDR2 having 100% sequence identity with sequence SEQ ID NO: 15 and L-CDR3 having 100% sequence identity with sequence SEQ ID NO: 9 in a VL having at least 85 % identity with the sequence set forth as SEQ ID NO: 37.

In some embodiments, the fusion protein of the invention comprises an anti-CCR8 antibody fragment, i.e. a CCR8 antibody binding fragment, and in particular an anti-CCR8 antibody fragment selected from the group consisting of a Fv, Fab, F(ab')2, Fab', dsFv, scFv, sc(Fv)2 and a diabody.

The glycosylation of the antibodies, or fragments thereof, of a fusion protein of the invention may in particular be altered in view of its fucose content.

Glycosylation of antibodies is typically N-linked. "*N-linked*" refers to the attachment of the carbohydrate moiety to the side chain of an asparagine residue. The tripeptide sequences asparagine-X-serine and asparagines-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site.

Removal of any carbohydrate moieties present on the antibody may be accomplished chemically or enzymatically. Chemical deglycosylation requires exposure of the antibody to the compound trifluoromethanesulfonic acid, or an equivalent compound. This treatment results in the cleavage of most or all sugars except the linking sugar (N-acetylglucosamine or N-acetylgalactosamine), while leaving the antibody intact. Chemical deglycosylation is described by Sojahr H. *et al.* (1987) and by Edge, AS. *et al.* (1981). Enzymatic cleavage of carbohydrate moieties on antibodies can be achieved by the use of a variety of endo-and exo-glycosidases as described by Thotakura, NR. et al. (1987).

More particularly, the glycosylation of the antibodies of the invention may be altered in that less than 65% of the glycan structures carried by the glycosylation site of the antibody comprise a fucose molecule.

Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation.

For example, EP 1176195 by Hang et al. describes a cell line with a functionally disrupted FUT8 gene, which encodes a fucosyl transferase, such that antibodies expressed in such a cell line exhibit hypofucosylation or are devoid of fucosyl residues.

Eureka Therapeutics further describes genetically engineered CHO mammalian cells capable of producing antibodies with altered mammalian glycosylation pattern devoid of fucosyl residues (http://www.eurekainc.com/a&boutus/companyoverview.html).

An antibody according to the invention is in particular devoid of fucosyl residues (i.e. afucosylated). In the present invention, to indicate that a given antibody is afucosylated (or that a fusion protein according to the invention comprises an afucosylated antibody or fragment thereof), the letter "A" is indicated after the name of said antibody. For example, antibody E201 is named E201A when afucosylated. Humanized afucosylated antibody E201 is accordingly named E201hA.

A fusion protein according to the invention comprises at least one IL-2 variant, this IL-2 variant being a Treg-specific IL-2 receptor antagonist.

The at least one IL-2 variant is preferably a human IL-2 variant.

In some particular embodiments, the IL-2 variant has at least 85% amino acid sequence identity with SEQ ID NO: 1 (wild-type human IL-2). In particular, said IL-2 variant may have at least 86%, 87%, 88%, 89%, 90%, 91%, 92%, 93%, 94%, 95%, 96%, 97%, 98% or 99% sequence identity with said sequence, even more preferably at least 95%, 96%, 97%, 98% or 99% 98% or 99% sequence identity with said sequence.

In some particular embodiments, the IL-2 variant has at least 85%, 86%, 87%, 88%, 89%, 90%, 91%, 92%, 93%, 94%, 95%, 96%, 97%, 98% or 99% sequence identity with any one of SEQ ID NO: 38, 39 or 40; preferably at least 95%, 96%, 97%, 98% or 99% 98% or 99% sequence identity with any one of SEQ ID NO: 38, 39 or 40; more preferably at least 85%, 86%, 87%, 88%, 89%, 90%, 91%, 92%, 93%, 94%, 95%, 96%, 97%, 98% or 99% sequence identity with SEQ ID NO: 38; even more preferably at least 95%, 96%, 97%, 98% or 99% 98% or 99% sequence identity with SEQ ID NO: 38.

An IL-2 variant according to the invention preferably comprises at least one amino acid deletion, more preferably at a position selected from S4, S5 or S6, the indicated positions being determined by alignment with SEQ ID NO: 1.

The IL-2 variant according to the invention in particular does not comprise any substitution at positions: 11, 13, 15, 18, 20, 22, 29, 30, 35, 37, 48, 68, 69, 71, 74, 75, 76, 80, 85, 86, 88, 92, 110, 126, 129, 130 and 133; 11, 13, 15, 18, 20, 22, 29, 30, 35, 37, 48, 68, 69, 71, 74, 75, 76, 80, 85, 86, 88, 92, 110, 125, 126, 129, 130 and 133 or at positions 4, 8, 10, 11, 13, 15, 18, 20, 22, 29, 30, 35, 37, 38, 42, 45, 48, 62, 67, 68, 69, 71, 74, 75, 76, 80, 85, 86, 88, 90, 92, 110, 125, 126, 128, 129, 130 and 133.

The Treg-specific IL-2 receptor antagonist in particular comprises the substitutions K9E, L12E, H16R, L19R, M23L, N26K, S87N, V91K, E95K, N119K and T123A;
and further comprises:
(i) the substitutions S127K and Y31P; or
(ii) the substitutions K49Q, E52S, R81E, D84N, T131R and L132S; or
(iii) the substitution S127K;
the indicated positions being determined by alignment with wild-type human IL-2 set forth as sequence SEQ ID NO: 1.

Preferably, the Treg-specific IL-2 receptor antagonist comprises the substitutions K9E, L12E, H16R, L19R, M23L, N26K, S87N, V91K, E95K, N119K and T123A;
and further comprises the substitutions S127K and Y31P,
the indicated positions being determined by alignment with wild-type human IL-2 set forth as sequence SEQ ID NO: 1.

Herein, an IL-2 receptor antagonist comprising the substitutions K9E, L12E, H16R, L19R, M23L, N26K, S87N, V91K, E95K, N119K and T123A, as well as the substitutions S127K and Y31P, the indicated positions being determined by alignment with wild-type human IL-2 set forth as sequence SEQ ID NO: 1, is termed "V5" or "IL-2V5".

In the various embodiments, the IL-2 variant may comprise at least one additional amino acid mutation (insertion, deletion, substitution) or not. In some embodiments, the IL-2 variant does not comprise additional amino acid substitutions, and in particular does not comprise additional amino acid mutations.

The IL-2 variant according to the invention may in particular comprise, and more particularly consists in, the sequence selected from the group consisting of SEQ ID NO: 38, SEQ ID NO: 39 and SEQ ID NO: 40, and preferably SEQ ID NO: 38.

A fusion protein according to the invention comprises at least one IL-2 variant. In particular, a fusion protein according to the invention comprises one or two IL-2 variants.

In a fusion protein of the invention, the IL-2 variant(s) is fused to the C-terminus of the antibody heavy chain, which means that the N-terminus of the IL-2 variant is fused to the C-terminus of the antibody heavy chain. The IL-2 variant may be fused to the antibody directly or via a linker or spacer that is used to physically separate two protein domains. Suitable linkers are known in the art and include for example linkers comprising Glycine and Serine residues and others. An example of suitable linker comprises the sequence (GGGGS)n, wherein n is preferably no more than 10 (1, 2, 3, 4, 5, 6,7, 8, 9, 10). In some embodiments, the fusion protein comprises a linker of sequence (GGGGS)3, corresponding to SEQ ID NO: 41.

In some particular embodiments, the fusion protein according to the invention is derived from a whole antibody molecule, preferably whole human or humanized antibody molecule, such as human or humanized IgG1 and IgG4 and the N-terminus of the IL-2 variant is fused to the C-terminus of the antibody heavy chain via a linker such as (GGGGS)n, more preferably (GGGGS)3.

In a particular embodiment, the fusion protein according to the invention comprises an anti-CCR8 antibody, or a fragment thereof, and at least one, in particular one or two, IL-2 variant which is a Treg-specific IL-2 receptor antagonist;
wherein the N-terminus of the at least one IL-2 variant is fused to the C-terminus of the antibody heavy chain;
the anti-CCR8 antibody or fragment thereof comprising:
   (i) a heavy chain comprising:
      - a H-CDR1 having the sequence set forth as SEQ ID NO: 3;
      - a H-CDR2 having a sequence selected from the group consisting of the sequences set forth as SEQ ID NO: 4 and SEQ ID NO: 11, and is in particular SEQ ID NO: 11; and
      - a H-CDR3 having a sequence selected from the group consisting of the sequences set forth as SEQ ID NO: 5 and SEQ ID NO: 12, and is in particular SEQ ID NO: 12;
         and
   (ii) a light chain comprising:
      - a L-CDR1 having a sequence selected from the group consisting of the sequences set forth as SEQ ID NO: 7 and SEQ ID NO: 14, and is in particular SEQ ID NO: 14;
      - a L-CDR2 having a sequence selected from the group consisting of the sequences set forth as SEQ ID NO: 8 and SEQ ID NO: 15, and is in particular SEQ ID NO: 15; and
      - a L-CDR3 having a sequence selected from the group consisting of the sequences set forth as SEQ ID NO: 9 and SEQ ID NO: 16, and is in particular SEQ ID NO: 16;
the at least one IL-2 variant comprising, and in particular consisting in, the sequence set forth as SEQ ID NO: 38,
the antibody being in particular afucosylated.

The fusion protein may comprise an additional moiety and/or may be further modified. The invention encompasses immunocytokines having one or more chemical modifications into one or more amino acid residues, peptide bonds, N-and/or C-terminal ends, as long as the modified immmunocytokine is functional. These modifications which are introduced into the fusion protein by the conventional methods known to those skilled in the art, include, in a non-limiting manner: coupling to a molecule or agent of interest, for example PEG (pegylation) to increase the biological (e.g., serum) half-life of the immunocytokine; substitution of a natural amino acid with a non-proteinogenic amino acid (D amino acid or amino acid analog); modification of the peptide bond, in particular with a bond of the retro or retro-inverso type or a bond different from the peptide bond; the cyclization, and the addition of a chemical group to the amino acid side chain or the N-and/or C-terminal end(s) of the immunocytokine, in particular for coupling to a molecule or agent of interest to the immunocytokine.

In some embodiments the IL-2 variant is provided in a form where it is associated with at least an agent of interest, for example in the form of a complex such as a molecular complex or a particle or a conjugate. The agent of interest includes with no limitation any therapeutic agent including a cell such as patient's Chimeric Antigen Receptor (CAR) T-cell.

The term immunocytokine as used herein encompasses the different forms of immunocytokines disclosed herein, such as an immunocytokine, modified or not, associated or not with at least an agent of interest in the form of a complex or conjugate as disclosed above.

The immunocytokine according to the invention can be made by routine techniques in the art, in particular by expression of a recombinant DNA in a suitable cell system (eukaryotic or prokaryotic) and screened for activity (i.e. capable of inducing CD25 endocytosis and/or inducing Treg apoptosis) using the assays described herein or other similar assays.

### Pharmaceutical composition and therapeutic use

A protein fusion, or immunocytokine, according to the invention may be incorporated in a pharmaceutical composition.

Accordingly, the invention also relates to a pharmaceutical composition comprising, in a pharmaceutically acceptable medium, at least a fusion protein according to the invention.

A "*pharmaceutically acceptable medium*" refers to a non-toxic material that is compatible with a biological system such as a cell, a cell culture, a tissue or an organism. In particular, a pharmaceutical composition is said to comprise a "*pharmaceutically acceptable medium*" if its administration can be tolerated by a recipient patient. Sterile phosphate-buffered saline is one example of a pharmaceutically acceptable carrier. Other suitable carriers are well-known to those in the art. (See, e.g., Gennaro (ed.), Remington's Pharmaceutical Sciences (Mack Publishing Company, 19th ed. 1995).) Formulations may further include one or more excipients, preservatives, solubilizers, buffering agents, albumin to prevent protein loss on vial surfaces, etc. The form of the pharmaceutical composition of the invention, the route of administration, the dosage and the regimen naturally depend upon the condition to be treated, the severity of the illness, the age, weight, and sex of the patient, etc.

In particular, the pharmaceutical composition contains vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or suspensions. The solution or suspension may comprise additives which are compatible with the active agent. In all cases, the form must be sterile and must be fluid to the extent that easy syringe ability exists. It must be stable under the conditions of manufacture and storage and must be preserved against the contaminating action of microorganisms, such as bacteria and fungi. An example of an appropriate solution is a buffer, such as phosphate buffered saline (PBS) or Ringer lactate.

The pharmaceutical composition is formulated for administration by a number of routes, including but not limited to oral, parenteral and local. The pharmaceutical vehicles are those appropriate to the planned route of administration, which are well known in the art. Compositions can take the form of one or more dosage units.

In some particular embodiments, the pharmaceutical composition is a liquid preparation, preferably comprising a concentrated liquid formulation of the immunocytokine. In some other particular embodiments, the pharmaceutical composition is a lyophilized preparation.

The pharmaceutical composition comprises a therapeutically effective amount of the immunocytokine. In the context of the invention a therapeutically effective amount refers to a dose sufficient for reversing, alleviating or inhibiting the progress of the disorder or condition to which such term applies, or reversing, alleviating or inhibiting the progress of one or more symptoms of the disorder or condition to which such term applies. The term "effective dose" or "effective dosage" is defined as an amount sufficient to achieve, or at least partially achieve, the desired effect. A therapeutically effective amount is sufficient to show a positive medical response in the individual to whom it is administered. A positive medical response refers to the reduction of subsequent (preventive treatment) or established (therapeutic treatment) disease symptoms. The positive medical response comprises a partial or total inhibition of the symptoms of the disease. A positive medical response can be determined by measuring various objective parameters or criteria such as objective clinical signs of the disease and/or the increase of survival. A medical response to the composition according to the invention can be readily verified in appropriate animal models of the disease which are well-known in the art.

The effective dose is determined and adjusted depending on factors such as the composition used, the route of administration, the physical characteristics of the individual under consideration such as sex, age and weight, concurrent medication, and other factors, that those skilled in the medical arts will recognize. The effective dose can be determined by standard clinical techniques. In addition, in vivo and/or in vitro assays may optionally be employed to help predict optimal dosage ranges.

In some particular embodiments, the therapeutically effective amount of the immunocytokine is at least 0.05 mg/kg.

In some embodiments, the pharmaceutical composition comprises another active agent wherein said active agent is a pharmaceutical agent or therapeutic capable of preventing, treating or ameliorating cancer in humans or animals. The active agent may be a protein including an antibody, an oligonucleotide including an antisense oligonucleotide, peptide nucleic acid (PNA), small interfering RNA, locked nucleic acids (LNA), phosphorodiamidate morpholino oligonucleotides (PMO) and decoy DNA molecule, a plasmid, an aptamer including DNA, RNA or peptide aptamer, a small or large chemical drug, or mixtures thereof. In particular, the active agent may be an immunomodulatory agent such as immune checkpoint inhibitor. The active agent may also be an anticancer agent or a tumor antigen.

A protein fusion of the invention and a pharmaceutical composition of the invention are used for treating cancer.

Accordingly, the present invention also relates to a fusion protein and/or a pharmaceutical composition of the invention for use in the treatment of cancer.

The invention also relates to the use of a fusion protein and/or a pharmaceutical composition of the invention for the manufacture of a medicament for treating cancer.

The invention also relates to a method for treating cancer, comprising the administration to an individual in need thereof, of a fusion protein and/or a pharmaceutical composition according to the invention.

The immunocytokine according to the invention are administered to the patient and the tumor-infiltrating Tregs, in particular tumor-associated Tregs, are inhibited or eliminated *in vivo* in the patient, unleashing immune cells (B, NK, CD4+ or CD8+ T cells; DC; macrophages and others) from Treg suppression. The immunocytokines according to the invention may advantageously be used to selectively deplete tumor-infiltrating Tregs, in particular tumor-associated Tregs, by depriving them from IL-2 while leaving effector immune cells, in particular NK cells, CD4+ and CD8+ T cells untouched and allowing their expansion. In particular embodiments, the immunocytokines according to the invention are used to selectively deplete tumor-infiltrating Tregs, in particular tumor-associated Tregs. In particular embodiments, the immunocytokines are used to stimulate an anti-tumoral CD8+ T-cell response.

The term "cancer" according to the invention comprises leukemias, seminomas, melanomas, teratomas, lymphomas, non-Hodgkin lymphoma, neuroblastomas, gliomas, adenocarcinoma, mesothelioma (including pleural mesothelioma, peritoneal mesothelioma, pericardial mesothelioma and end stage mesothelioma), rectal cancer, endometrial cancer, thyroid cancer (including papillary thyroid carcinoma, follicular thyroid carcinoma, medullary thyroid carcinoma, undifferentiated thyroid cancer, multiple endocrine neoplasia type 2A, multiple endocrine neoplasia type 2B, familial medullary thyroid cancer, pheochromocytoma and paraganglioma), skin cancer (including malignant melanoma, basal cell carcinoma, squamous cell carcinoma, Kaposi's sarcoma, keratoacanthoma, moles, dysplastic nevi, lipoma, angioma and dermatofibroma), nervous system cancer, brain cancer (including astrocytoma, medulloblastoma, glioma, lower grade glioma, ependymoma, germinoma (pinealoma), glioblastoma multiform, oligodendroglioma, schwannoma, retinoblastoma, congenital tumors, spinal cord neurofibroma, glioma or sarcoma), skull cancer (including osteoma, hemangioma, granuloma, xanthoma or osteitis deformans), meninges cancer (including meningioma, meningiosarcoma or gliomatosis), head and neck cancer (including head and neck squamous cell carcinoma and oral cancer (such as, e.g., buccal cavity cancer, lip cancer, tongue cancer, mouth cancer or pharynx cancer)), lymph node cancer, gastrointestinal cancer, liver cancer (including hepatoma, hepatocellular carcinoma, cholangiocarcinoma, hepatoblastoma, angiosarcoma, hepatocellular adenoma and hemangioma), colon cancer, stomach or gastric cancer, esophageal cancer (including squamous cell carcinoma, larynx, adenocarcinoma, leiomyosarcoma or lymphoma), colorectal cancer, intestinal cancer, small bowel or small intestines cancer (such as, e.g., adenocarcinoma lymphoma, carcinoid tumors, Kaposi's sarcoma, leiomyoma, hemangioma, lipoma, neurofibroma or fibroma), large bowel or large intestines cancer (such as, e.g., adenocarcinoma, tubular adenoma, villous adenoma, hamartoma or leiomyoma), pancreatic cancer (including ductal adenocarcinoma, insulinoma, glucagonoma, gastrinoma, carcinoid tumors or vipoma), ear, nose and throat (ENT) cancer, breast cancer (including HER2-enriched breast cancer, luminal A breast cancer, luminal B breast cancer and triple negative breast cancer), cancer of the uterus (including endometrial cancer such as endometrial carcinomas, endometrial stromal sarcomas and malignant mixed Müllerian tumors, uterine sarcomas, leiomyosarcomas and gestational trophoblastic disease), ovarian cancer (including dysgerminoma, granulosa-theca cell tumors and Sertoli-Leydig cell tumors), cervical cancer, vaginal cancer (including squamous-cell vaginal carcinoma, vaginal adenocarcinoma, clear cell vaginal adenocarcinoma, vaginal germ cell tumors, vaginal sarcoma botryoides and vaginal melanoma), vulvar cancer (including squamous cell vulvar carcinoma, verrucous vulvar carcinoma, vulvar melanoma, basal cell vulvar carcinoma, Bartholin gland carcinoma, vulvar adenocarcinoma and erythroplasia of Queyrat), genitourinary tract cancer, kidney cancer (including clear renal cell carcinoma, chromophobe renal cell carcinoma, papillary renal cell carcinoma, adenocarcinoma, Wilms tumor, nephroblastoma, lymphoma or leukemia), adrenal cancer, bladder cancer, urethra cancer (such as, e.g., squamous cell carcinoma, transitional cell carcinoma or adenocarcinoma), prostate cancer (such as, e.g., adenocarcinoma or sarcoma) and testis cancer (such as, e.g., seminoma, teratoma, embryonal carcinoma, teratocarcinoma, choriocarcinoma, sarcoma, interstitial cell carcinoma, fibroma, fibroadenoma, adenomatoid tumors or lipoma), lung cancer (including small cell lung carcinoma (SCLC), non-small cell lung carcinoma (NSCLC) including squamous cell lung carcinoma, lung adenocarcinoma (LUAD), and large cell lung carcinoma, bronchogenic carcinoma, alveolar carcinoma, bronchiolar carcinoma, bronchial adenoma, lung sarcoma, chondromatous hamartoma and pleural mesothelioma), sarcomas (including Askin's tumor, sarcoma botryoides, chondrosarcoma, Ewing's sarcoma, malignant hemangioendothelioma, malignant schwannoma, osteosarcoma and soft tissue sarcomas), soft tissue sarcomas (including alveolar soft part sarcoma, angiosarcoma, cystosarcoma phyllodes, dermatofibrosarcoma protuberans, desmoid tumor, desmoplastic small round cell tumor, epithelioid sarcoma, extraskeletal chondrosarcoma, extraskeletal osteosarcoma, fibrosarcoma, gastrointestinal stromal tumor (GIST), hemangiopericytoma, hemangiosarcoma, Kaposi's sarcoma, leiomyosarcoma, liposarcoma, lymphangiosarcoma, lymphosarcoma, malignant peripheral nerve sheath tumor (MPNST), neurofibrosarcoma, plexiform fibrohistiocytic tumor, rhabdomyosarcoma, synovial sarcoma and undifferentiated pleomorphic sarcoma, cardiac cancer (including sarcoma such as, e.g., angiosarcoma, fibrosarcoma, rhabdomyosarcoma or liposarcoma, myxoma, rhabdomyoma, fibroma, lipoma and teratoma), bone cancer (including osteogenic sarcoma, osteosarcoma, fibrosarcoma, malignant fibrous histiocytoma, chondrosarcoma, Ewing's sarcoma, malignant lymphoma and reticulum cell sarcoma, multiple myeloma, malignant giant cell tumor chordoma, osteochronfroma, osteocartilaginous exostoses, benign chondroma, chondroblastoma, chondromyxoid fibroma, osteoid osteoma and giant cell tumors), hematologic and lymphoid cancer, blood cancer (including acute myeloid leukemia, chronic myeloid leukemia, acute lymphoblastic leukemia, chronic lymphocytic leukemia, myeloproliferative diseases, multiple myeloma and myelodysplasia syndrome), Hodgkin's disease, non-Hodgkin's lymphoma and hairy cell and lymphoid disorders, and the metastases thereof. The lymphoma may be for example a T cell lymphoma such as anaplastic T cell lymphoma.

The cancer may be a solid or liquid cancer, in particular chosen from any one of the cancer disclosed herein.

A cancer according to the invention may in particular be selected from the group consisting of Melanoma; renal cancer, in particular Renal Cell Carcinoma; Colorectal Cancer; liver cancer, in particular Hepatocellular Carcinoma; lung cancer, in particular Non-Small Cell Lung Cancer; mesothelioma, in particular Malignant Pleural Mesothelioma; Esophageal Cancer, in particular esophageal squamous cell carcinoma or adenocarcinoma; Head and Neck cancer, in particular Head and Neck Squamous Cell Carcinoma; urothelial cancer, in particular urothelial carcinoma; Lymphoma, in particular Primary Hodgkin Lymphoma or Large B Cell Lymphoma; Gastric Cancer, in particular gastric or gastroesophageal junction adenocarcinoma; Cervical Cancer; Merkel Cell Carcinoma; Endometrial cancer, in particular endometrial Carcinoma; skin cancer, in particular cutaneous squamous cell carcinoma; Breast cancer, in particular Triple Negative Breast Cancer or Breast invasive Carcinoma; Pancreatic cancer, in particular pancreatic Adenocarcinoma; Thymoma; Prostate cancer, in particular prostate adenocarcinoma; Ovarian cancer, in particular ovarian serous cystadenocarcinoma; Thyroid cancer, in particular thyroid carcinoma, and Sarcoma.

The immunocytokine or pharmaceutical composition of the present invention is generally administered according to known procedures, at dosages and for periods of time effective to induce a therapeutic effect in the individual. The immunocytokine or pharmaceutical composition according to the invention may be administered by any convenient route, such as in a non-limiting manner by infusion or bolus injection, by absorption through epithelial or mucocutaneous linings (e.g., oral mucosa, rectal and intestinal mucosa, etc.), inhalation, transdermal application or intratumoral administration. The administration can be systemic, local or systemic combined with local; systemic includes parenteral and oral, and local includes local, loco-regional and intra-tumoral. Systemic administration is preferably parenteral such as subcutaneous (SC), intramuscular (IM), intravascular such as intravenous (IV) or intraarterial; intraperitoneal (IP); intradermal (ID), epidural or else. The parenteral administration is advantageously by injection or perfusion. In some particular embodiments, the administration is parenteral and/or intratumoral, preferably intravascular such as intravenous (IV), intratumoral or IV combined with intratumoral.

By "*therapeutic regimen*" is meant the pattern of treatment of an illness, e.g., the pattern of dosing used during therapy. A therapeutic regimen may include an induction regimen and a maintenance regimen. The phrase "*induction regimen*" or "*induction period*" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "*loading regimen*"*,* which may include administering a greater dose of the drug than a physician would employ during a maintenance regimen, administering a drug more frequently than a physician would administer the drug during a maintenance regimen, or both. The phrase "*maintenance regimen*" or "*maintenance period*" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

In some embodiments, the immunocytokine or pharmaceutical composition according to the invention is used for the prevention or treatment of cancer in a human.

The immunocytokine or composition of the invention is advantageously used in combination with another cancer therapy. In particular, the immunocytokine and/or pharmaceutical composition of the invention may be administered in combination with targeted therapy; immunotherapy such as immune checkpoint therapy and immune checkpoint inhibitor, co-stimulatory antibodies, and CAR-T cell therapy; anticancer agents including therapeutic agents (chemotherapy) and vaccines against cancer and/or radiotherapy. The combined therapies may be separate, simultaneous, and/or sequential.

Immune checkpoint therapy such as checkpoint inhibitors include, but are not limited to programmed death-1 (PD-1) inhibitors, programmed death ligand-1 (PD-L1) inhibitors, programmed death ligand-2 (PD-L2) inhibitors, lymphocyte-activation gene 3 (LAG-3) inhibitors, T-cell immunoglobulin and mucin-domain containing protein 3 (TIM-3) inhibitors, T cell immunoreceptor with Ig and ITIM domains (TIGIT) inhibitors, B- and T-lymphocyte attenuator (BTLA) inhibitors, V-domain Ig suppressor of T-cell activation (VISTA) inhibitors, Indoleamine 2,3-dioxygenase (IDO) inhibitors, killer immunoglobulin-like receptors (KIR) inhibitors, KIR2L3 inhibitors, KIR3DL2 inhibitors and carcinoembryonic antigen-related cell adhesion molecule 1 (CEACAM-1) inhibitors. In particular, checkpoint inhibitors include antibodies anti-PD1, anti-PD-L1, anti-CTLA-4, anti-TIM-3, anti-LAG3. Co-stimulatory antibodies deliver positive signals through immune-regulatory receptors including but not limited to ICOS, CD137, CD27, OX-40 and GITR.

Examples of anti-PD1 antibodies include, but are not limited to, nivolumab, cemiplimab (REGN2810 or REGN-2810), tislelizumab (BGB-A317), spartalizumab (PDR001 or PDR-001), ABBV-181, JNJ-63723283, BI 754091, MAG012, TSR-042, AGEN2034, pidilizumab, nivolumab (ONO-4538, BMS-936558, MDX1106, GTPL7335 or Opdivo), pembrolizumab (MK-3475, MK03475, lambrolizumab, SCH-900475 or Keytruda) and antibodies described in International patent applications WO2004004771, WO2004056875, WO2006121168, WO2008156712, WO2009014708, WO2009114335, WO2013043569 and WO2014047350.

Examples of anti-PD-L1 antibodies include, but are not limited to, LY3300054, atezolizumab, durvalumab and avelumab.

Example of anti-VISTA antibodies are described in US patent application US20130177557.

Example of inhibitors of the LAG3 receptor are described in US patent US5,773,578.

Example of KIR inhibitor is IPH4102 targeting KIR3DL2.

Example of anti-CTLA-4 antibodies can for example be selected from the group consisting of ipilimumab, tremelimumab and quavonlimab.

Targeted therapy are drugs designed to interfere with specific molecules necessary for tumor growth and progression. For example, therapeutic monoclonal antibodies target specific antigens found on the cell surface, such as transmembrane receptors or extracellular growth factors. In some cases, monoclonal antibodies are conjugated to radio-isotopes or toxins to allow specific delivery of these cytotoxic agents to the intended cancer cell target. Small molecules can penetrate the cell membrane to interact with targets inside a cell. Small molecules are usually designed to interfere with the enzymatic activity of the target protein such as for example proteasome inhibitor, tyrosine kinase or cyclin-dependent kinase inhibitor, histone deacetylase inhibitor. Targeted therapy may also use cytokines. Examples of such targeted therapy include with no limitations: Adotrastuzumab emtansine (HER2), Afatinib (EGFR (HER1/ERBB1), HER2), Aldesleukin (Proleukin), alectinib (ALK), Alemtuzumab (CD52), axitinib (kit, PDGFRbeta, VEGFR1/2/3), Belimumab (BAFF), Belinostat (HDAC), Bevacizumab (VEGF ligand), Blinatumomab (CD19/CD3), bortezomib (proteasome), Brentuximab vedotin (CD30), bosutinib (ABL), brigatinib (ALK), cabozantinib (FLT3, KIT, MET, RET, VEGFR2), Canakinumab (IL-1 beta), carfilzomib (proteasome), ceritinib (ALK), Cetuximab (EGFR), cofimetinib (MEK), Crizotinib (ALK, MET, ROS1), Dabrafenib (BRAF), Daratumumab (CD38), Dasatinib (ABL), Denosumab (RANKL), Dinutuximab (B4GALNT1 (GD2)), Elotuzumab (SLAMF7), Enasidenib (IDH2), Erlotinib (EGFR), Everolimus (mTOR), Gefitinib (EGFR), Ibritumomab tiuxetan (CD20), Sonidegib (Smoothened), Sipuleucel-T, Siltuximab (IL-6), Sorafenib (VEGFR, PDGFR, KIT, RAF),(Tocilizumab (IL-6R), Temsirolimus (mTOR), Tofacitinib (JAK3), Trametinib (MEK), Tositumomab (CD20), Trastuzumab (HER2), Vandetanib (EGFR), Vemurafenib (BRAF), Venetoclax (BCL2), Vismodegib (PTCH, Smoothened), Vorinostat (HDAC), Ziv-aflibercept (PIGF, VEGFA/B).

In some embodiments, the immunocytokine and/or pharmaceutical composition of the invention is administered to the patient in combination with chemotherapy. As used herein, the term "*chemotherapy*" has its general meaning in the art and refers to the treatment that consists in administering to the patient a chemotherapeutic agent. Chemotherapeutic agents include, but are not limited to alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g. , calicheamicin, especially calicheamicin gammall and calicheamicin omegall ; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolinodoxorubicin and deoxy doxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; methylhydrazine derivatives including N-methylhydrazine (MIH) and procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-1 1); topoisomerase inhibitor RFS 2000; difluoromethylomithine (DMFO); retinoids such as retinoic acid; capecitabine; anthracyclines, nitrosoureas, antimetabolites, epipodophylotoxins, enzymes such as L-asparaginase; anthracenediones; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide; and pharmaceutically acceptable salts, acids or derivatives of any of the above.

In some embodiments, the immunocytokine and/or pharmaceutical composition of the invention is administered to the patient in combination with radiotherapy. Suitable examples of radiation therapies include, but are not limited to external beam radiotherapy (such as superficial X-rays therapy, orthovoltage X-rays therapy, megavoltage X-rays therapy, radiosurgery, stereotactic radiation therapy, Fractionated stereotactic radiation therapy, cobalt therapy, electron therapy, fast neutron therapy, neutron-capture therapy, proton therapy, intensity modulated radiation therapy (IMRT), 3-dimensional conformal radiation therapy (3D-CRT) and the like); brachytherapy; unsealed source radiotherapy; tomotherapy; and the like. Gamma rays are another form of photons used in radiotherapy. Gamma rays are produced spontaneously as certain elements (such as radium, uranium, and cobalt 60) release radiation as they decompose, or decay. In some embodiments, radiotherapy may be proton radiotherapy or proton minibeam radiation therapy. Proton radiotherapy is an ultra-precise form of radiotherapy that uses proton beams (Prezado Y, Jouvion G, Guardiola C, Gonzalez W, Juchaux M, Bergs J, Nauraye C, Labiod D, De Marzi L, Pouzoulet F, Patriarca A, Dendale R. Tumor Control in RG2 Glioma-Bearing Rats: A Comparison Between Proton Minibeam Therapy and Standard Proton Therapy. Int J Radiat Oncol Biol Phys. 2019 Jun 1;104(2):266-271. doi: 10.1016/j ijrobp.2019.01.080; Prezado Y, Jouvion G, Patriarca A, Nauraye C, Guardiola C, Juchaux M, Lamirault C, Labiod D, Jourdain L, Sebrie C, Dendale R, Gonzalez W, Pouzoulet F. Proton minibeam radiation therapy widens the therapeutic index for high-grade gliomas. Sci Rep. 2018 Nov 7;8(1):16479. doi: 10.1038/s41598-018-34796-8). Radiotherapy may also be FLASH radiotherapy (FLASH-RT) or FLASH proton irradiation. FLASH radiotherapy involves the ultra-fast delivery of radiation treatment at dose rates several orders of magnitude greater than those currently in routine clinical practice (ultra-high dose rate) (Favaudon V, Fouillade C, Vozenin MC. The radiotherapy FLASH to save healthy tissues. Med Sci (Paris) 2015 ; 31 : 121-123. DOI: 10.1051/medsci/20153102002); Patriarca A., Fouillade C. M., Martin F., Pouzoulet F., Nauraye C., et al. Experimental set-up for FLASH proton irradiation of small animals using a clinical system. Int J Radiat Oncol Biol Phys, 102 (2018), pp. 619-626. doi: 10.1016/j.ijrobp.2018.06.403. Epub 2018 Jul 11).

In some particular embodiments, the immunocytokine and/or pharmaceutical composition according to the invention is administered in combination with at least one immune check-point inhibitor; in particular at least one selected from the group consisting of anti-PD-1, anti-PDL-1 or anti-CTLA-4 monoclonal antibodies.

The invention will be further illustrated by the following figures and examples. However, these examples and figures should not be interpreted in any way as limiting the scope of the present invention.

### EXAMPLES

The antibody of the fusion protein according to the invention implemented in the following examples is humanized antibody E201 as defined above.

### Example 1: Immunocytokines of the invention and CCR8 internalization

Expanded Tregs were treated with anti-TNFR2 antibody (3µg/mL) for 48h to induce CCR8 expression.

Cells were counted and resuspended at 1 million/mL in X-vivo medium. 300-400000 Cells were seeded in 24 well plate and incubated for 1h at 37°C with 5nM of different compounds:
- a fusion protein according to the invention (comprising one cytokine, i.e. monovalent): E201hA-IL2V51x;
- a fusion protein according to the invention (comprising two cytokines, i.e. bivalent): E201hA-IL2V52x.
- an afucosylated anti-CCR8 antibody (E201hA) as defined herein but not fused to a cytokine (outside of the invention).

After that, cells were seeded in 12mm coverslips precoated with Poly-L-Lysine (Sigma) for 30 min at 37°C. Cells were fixed with PFA 4% and stored at 4°C until the moment of the staining.

Staining and mounting: Cells were permeabilized for 30 min at room temperature with PBS/ 0.2% Bovine Serum Albumin/ 0.05% Saponin. Cells were then incubated for 1 h at room temperature with mouse anti-CD25 BV421 (1/50) in BSA/saponin buffer, washed three times with PBS/ 0.2% BSA/ 0.05% Saponin and incubated in the dark for 30 min with secondary donkey anti-human IgG AF488 antibody (1/200).

After washing with PBS/0.2% BSA/0.05% Saponin and PBS, coverslips were soaked three times in PBS, three times in water, mounted on slides with 4-6 µL of Fluoromount G and dried overnight in the dark before microscope acquisition.

Microscope and Image Analysis: a single cell z-plane was acquired using a confocal microscope LSM900 (Zeiss) with 63X oil Objective and pixel size around 55 nm. Laser: 405, 488, 647 nm.

Image analysis: Quantification was done using the Fiji program. Briefly, the cytosolic and the total cell surface was defined and drawn manually in each cell and the fluorescent integrated density for the Fc channel was collected. The percentage of the internalized compound was calculated as: fluorescent Int Density Fc cytosolic /Int Density Fc total cell.

The results obtained are illustrated in Figure 1.

These results show that immunocytokines of the invention have an enhanced membrane retention compared with the same antibody in its standard form (i.e. devoid of cytokine(s)).

### Example 2: Reporter ADCC assay with immunocytokines of the invention

To evaluate ADCC of immunocytokines according to the invention, a reporter assay with HUT78 as target cells and Jurkat-Lucia^{™} NFAT-CD16 cells (Invivogen) as effector cells was used.

100 000 HUT78 cells expressing CCR8 were incubated with different doses (0.1 nM, 0.5 nM, 2.5 nM or 12.5 nM) of immunocytokines according to the invention, standard antibody or isotype control for 1 hour at 37°C, 5%CO₂ in IMDM 10% FBS:
- isotype control (control): IgG1
- standard antibody (comparative): E201hA (humanized (h) and afucosylated (A) antibody E201);
- a fusion protein according to the invention (comprising two cytokines, i.e. bivalent); E201hA-IL2V52x.
- a fusion protein according to the invention (comprising one cytokine, i.e. monovalent); E201hA-IL2V51x.

200 000 Jurkat-Lucia^{™} NFAT-CD16 cells resuspended in IMDM 10% FBS were added, and cells were incubated at 37°C, 5% CO₂ for additional 6 hours. Supernatant was collected and mixed with Quanti Luc 4 Lucia/Gaussia (Invivogen) for luminescence measurement with Varioskan (Thermo Scientific).

The results obtained are represented in Figure 2.

The results show that immunocytokines of the invention, comprising one or two cytokine(s), present a significantly superior ADCC activity compared with the same antibody in its standard form (i.e. devoid of cytokine(s)).

### Example 3: NK ADCC assay of immunocytokines of the invention

Human PBMCs were isolated from whole blood of healthy donors by Lymphoprep (Stemcell Technologies) gradient centrifugation in 50 mL Blood-Sep-Filter tubes (Dacos). After centrifugation (800g, 15 min) the PBMC layer was removed, and cells were washed with RPMI 1640 (Thermo Fisher Scientific) twice.

Human Treg cells were isolated from PBMCs in two steps: first, CD25+ cells were isolated with CD25 MicroBeads II kit (Miltenyi Biotec) from total PBMCs. Then Tregs (CD4+CD8-CD127-CD25hi) were FACS sorted from the CD25+ cells using CytoFLEX (Beckman Coulter).

Tregs were then expanded *in vitro* by culturing them for 18 days in complete X-Vivo medium (Lonza) supplemented with 1% Pen/Strep, 1% MEM non-essential aminoacids, 10% decomplemented human serum, 1% b-mercaptoethanol, 300 iU/mL of Proleukin and anti-CD3/anti-CD28 beads (Miltenyi Biotec).

NK cells were isolated from whole blood of healthy donors using MACSxpress LRSC NK Cell Isolation Kit, human (Miltenyi Biotec) following instructions from the provider. After magnetic depletion, NK-cells were collected counted and activated using NK Cell Activation Expansion kit (Miltenyi Biotec), 500 iU/mL Proleukin and 140 iU/mL IL-15 in NK MACS Medium (Miltenyi Biotec) containing 5% Human Serum (Sigma), 1% penicillin/streptomycin (Thermo Fisher Scientific), NK MACS supplement 100X (Miltenyi Biotec) at 37°C, 5%CO₂.

10 000 expanded Tregs (target cells) were stained with 0.5 µM of CellTrace Violet (Life technologies) for 20 minutes in PBS at 37°C, 5%CO₂. Cells were washed, resuspended at 200 000/ml in complete RPMI 1640 and seeded at 50 µL/ well (10 000 Tregs). Cells were then incubated for 30 minutes with different dose (0,008 nM, 0.04 nM or 0.2 nM) of compounds 100 µL/ well (2X) at 37°C, 5%CO₂:
- isotype control (control): IgG1
- standard antibody (comparative): E201hA
- a fusion protein according to the invention (comprising two cytokines, i.e. bivalent): E201hA-IL2V52x
- negative control immunocytokine devoid of ADCC activity (comprising two cytokines and the E201h antibody in an Fc-silenced IgG1 backbone carrying the L234A and L235A mutations): E201h-LALA-IL2V52x, comparative.

Cells were centrifuged and supernatant discarded to remove excess compounds. Wells were resuspended in 150 µL complete RPMI 1640. NK-cells were counted, resuspended at 1 000 000/ml in complete NK MACS medium and added 50 µL/ well (Miltenyi Biotec) to Tregs cells with 150 nM of Proleukin.

After overnight incubation cells were washed and stained with 7-AAD (1/1000 in PBS) for 15 minutes at 4°C. 10 000 Countbright Absolute counting beads (Life technologies) were added for counting (10 µL/well) before FACS acquisition.

The results obtained are illustrated in Figure 3.

Immunocytokines of the invention demonstrate a higher ADCC activity than the standard antibodies in the NK-based ADCC assay.

### Example 4: Specificity of immunocytokines of the invention for CD25+CCR8+ cells but not for CD25+CCR8- cells

HUT78 cells expressing both CD25 and CCR8 were generated from conventional HUT78 cells via transduction with lentiviral vector bearing human CD25 receptor. HUT78 cells expressing only CD25 receptor were generated from conventional HUT78 cells in two steps. First, CCR8 knock-out was performed with CRISPR-Cas9 approach using two guide RNAs targeting human CCR8 gene. Second, HUT78 cells knock-out for CCR8 were transduced with lentiviral vector bearing human CD25 receptor.

Repoter ADCC assay was performed as described in example 2 with various concentrations (0,0008nM, 0.004nM, 0.02nM, 0.1nM, 0.5nM or 2.5nM) of the following compounds:
- isotype control (control): IgG1
- a fusion protein according to the invention (comprising one cytokine, i.e. monovalent): E201hA-IL2V51x.
- a fusion protein according to the invention (comprising two cytokines, i.e. bivalent): E201hA-IL2V52x.

The results obtained are illustrated in Figure 4.

A strong specificity of a fusion protein of the invention for CD25+CCR8+cells, versus CD25+CCR8- cells, is observed.

### Example 5: Immunocytokines of the invention and Tregs migration

Cell preparation: expanded Tregs were transduced (day 4-5) at MOI of 50 with lentivirus containing CCR8 fused to mCherry fluorescent protein (Vector Builder) and expanded for another 10-15 days. Cells were incubated with 5 nM of isotype control (control): IgG1, standard antibody (comparative): E201hA, or a fusion protein according to the invention (comprising two cytokines, i.e. bivalent): E201hA-IL2V52x for 1 hour at 37°C, stained with 1 µg/mL of Hoechst dye for 20 minutes at 37°C, washed, and resuspended at 3 million/mL in X-vivo medium (Lonza).

Chamber and CCL-1 gradient preparation: µ-Slide Chemotaxis slides (IBIDI) were coated with 10 µg/mL of fibronectin (Sigma) for 1 hour at room temperature and washed three times with PBS. 30,000 cells were seeded in the middle chamber and incubated for 45 minutes at 37°C. Next, both reservoirs adjacent to the middle chamber were filled with 65 µL chemoattractant CCL-1 500 ng/mL or complete X-vivo medium.

Microscopy acquisition and imaging quantification: Live imaging was performed using an LSM900 confocal microscope (Zeiss) with an epifluorescence module and a 20X objective. Each frame was acquired every 3 minutes at 37°C and 5% CO₂ for a total duration of 180 minutes. Lasers of 405, 568 nm and bright field channels were acquired. Quantification was done using the TrackMate plugin in the Fiji program. Briefly, each cell was tracked in each frame by following the nucleus staining through automatic thresholding. For each cell, the displacement from the initial position (time = 0 minutes), the mean speed, and the total displacement were determined.

The results obtained are illustrated in Figure 5.

The results show that while the standard anti-CCR8 antibody (i.e. anti-CCR8 antibody devoid of cytokine(s)) decreases Tregs migration, an immunocytokine of the invention completely blocks Tregs migration.

### Example 6: antitumor activity of immunocytokines of the invention

A549 (human lung carcinoma cell line) GFP cells were collected, resuspended in RPMI1640 10%FBS, 1% P/S and seeded in ultra-low attachment 96 well plate (Corning) at cell concentration 10 000/mL, 100 µL/ well (1 000 cells/well) at 37°C, 5%CO₂.

4 days later, 10 000 frozen PBMCs were added with 10 ng/mL anti-CD3 (BD, clone OKT3), 50 nM of tested compounds and 1000 or 2000 expanded Tregs (previously stimulated by anti-TNFR2 to promote CCR8 expression, 3 ug/mL for 48 hours).

Five situations were compared:
- spheroids with no PBMC (control);
- spheroids with PBMC (control);
- spheroids with PBMC and Tregs (control);
- spheroids with PBMC and Tregs and with standard antibody E201hA;
- spheroids with PBMC and Tregs and with an immunocytokine according to the invention E201hA-IL2V52x (fusion protein of the invention comprising two cytokines as defined above and an afucosylated antibody of the invention).

Intensity of green fluorescence was monitored for 6 days with CELLCYTE X Live Cell Imager (Cytena). Obtained data were analyzed using CELLCYTE X software.

The results obtained are illustrated in Figure 6.

The results show that compared to the standard antibody, the immunocytokine according to the invention unleashes anti-tumor T effector cells from Tregs control, which results in a significantly increased tumor spheroid death.

### Example 7: antitumor activity of immunocytokines of the invention

Fresh tumor samples obtained from patients with breast cancer were embedded into agarose and cut into 300 µm slices. Slices were incubated in complete RPMI in CO2 incubator overnight. Allogenic expanded Tregs (see Example 3) were added to the slices along with compounds at the final concentration of 5 µM and incubated for 6 days. After incubation with the compounds tumor slices were fixed in 4% PFA stained with Epcam-BV421 (Biolegend) Hoescht 33342 (ThermoScientific), CD45- APCCy7 (BD Bioscience) CD3-AF647 (Biolegend) Microscope and image analysis were performed on a single cell z-plane was acquired using a confocal microscope LSM900 (Zeiss) with 63X oil Objective

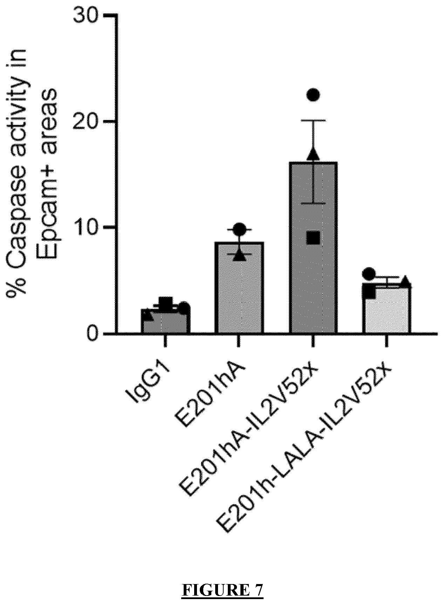

The results obtained are illustrated in Figure 7.

The results show that an immunocytokine of the invention triggers a significantly stronger apoptosis of cancer cells compared with the standard antibody.

### Description of the Sequences

**SEQ ID NO: 1**
   **WT Human IL-2**
**SEQ ID NO: 2**
   **Anti-CCR8 antibody VH sequence (antibody E201)** 
EVQLVETGGGLVQPKGSLKLSCAASGFTFNTNAMNWVRQAPGKGLEWVARIRTK
SNNYATYYADSVKDRFTISRDDSRRMLYLQMNNMKTEDTAMFYCVRGGYGISA
HNTMDYWGQGTSVTVSA
**SEQ ID NO: 3**
   **Anti-CCR8 antibody H-CDR1 (antibodies E201, E201h, E203 and E203h)**
   GFTFNTNA
**SEQ ID NO: 4**
   **Anti-CCR8 antibody H-CDR2 (antibody E201)**
   IRTKSNNYAT
**SEQ ID NO: 5**
   **Anti-CCR8 antibody H-CDR3 (antibody E201)**
   VRGGYGISAHNTMDYW
**SEQ ID NO: 6**
   **Anti-CCR8 antibody VL sequence (antibody E201)** 
DIVMTQSAPSVPVTPGESVSISCRSSKSLLHSNGNTYLYWFLQRPGQSPQLLIYRMS
NLASGVPDRFSGSGSGTAFTLRISRVEAEDVGVYYCMQHLEYPFTFGSGTKLEIK
**SEQ ID NO: 7**
   **Anti-CCR8 antibody L-CDR1 (antibodies E201 and E203)**
   KSLLHSNGNTY
**SEQ ID NO: 8**
   **Anti-CCR8 antibody L-CDR2 (antibodies E201 and E203)**
   RMS
**SEQ ID NO: 9**
   **Anti-CCR8 antibody L-CDR3 (antibodies E201 and E203)**
   MQHLEYPFT
**SEQ ID NO: 10**
   **Anti-CCR8 antibody VH sequence (antibody E201h)** 
EVQLVESGGGLVKPGGSLRLSCAASGFTFNTNAMNWVRQAPGKGLEWVARIRTK
SNQYATYYADSVKDRFTISRDDSRRSLYLQMNSLRAEDTAVYYCVRGGYGISAH
QTMDYWGQGTLVTVSS
**SEQ ID NO: 11**
   **Anti-CCR8 antibody H-CDR2 (antibody E201h)**
   IRTKSNQYAT
**SEQ ID NO: 12**
   **Anti-CCR8 antibody H-CDR3 (antibody E201h)**
   VRGGYGISAHQTMDYW
**SEQ ID NO: 13**
   **Anti-CCR8 antibody VL sequence (antibody E201h)** 
DIVMTQSPLSLPVTPGEPASISCRSSKSLLHSNAQTYLYWFLQKPGQSPQLLIYRLS
NLASGVPDRFSGSGSGTDFTLKISRVEAEDVGVYYCFQHLEYPFTFGGGTKLEIK
**SEQ ID NO: 14**
   **Anti-CCR8 antibody L-CDR1 (antibody E201h)**
   KSLLHSNAQTY
**SEQ ID NO: 15**
   **Anti-CCR8 antibody L-CDR2 (antibodies E201h and E203h)**
   RLS
**SEQ ID NO: 16**
   **Anti-CCR8 antibody L-CDR3 (antibody E201h)**
   FQBLEYPFT
**SEQ ID NO: 17**
   **Anti-CCR8 antibody VH sequence (antibody E202)** 
QVQLKESGPGLVQPSQSLSITCTVSGFSLSSYGVHWVRQSPGKGLEWLGVIWSGG
STDYNAAFISRLSISKDNSKSQVFFKMNSLQADDTAIYNCARNKVFNYYGSRYEV
MDYWGQGTSVTVSS
**SEQ ID NO: 18**
   **Anti-CCR8 antibody H-CDR1 (antibodies E202 and E202h)**
   GFSLSSYG
**SEQ ID NO: 19**
   **Anti-CCR8 antibody H-CDR2 (antibody E202)**
   IWSGGSTD
**SEQ ID NO: 20**
   **Anti-CCR8 antibody H-CDR3 (antibody E202)**
   CARNKVFNYYGSRYEVMDYW
**SEQ ID NO: 21**
   **Anti-CCR8 antibody VL sequence (antibody E202)** 
EILLTQSPAIIAASPGEKVTITCSASSSVVYMNWYQQKPGSSPKIWIYGISNLASGVP
TRFSGSGSGTSFSFTINSMEAEDVATYYCQQRTSSPPWTFGGGTKLEIK
**SEQ ID NO: 22**
   **Anti-CCR8 antibody L-CDR1 (antibody E202 and E202h)**
   SSVVY
**SEQ ID NO: 23**
   **Anti-CCR8 antibody L-CDR2 (antibody E202 and E202h)**
   GIS
**SEQ ID NO: 24**
   **Anti-CCR8 antibody L-CDR3 (antibody E202 and E202h)**
   QQRTSSPPWT
**SEQ ID NO: 25**
   **Anti-CCR8 antibody VH sequence (antibody E202h)** 
QVQLVESGGGVVQPGRSLRLSCAVSGFSLSSYGVHWVRQAPGKGLEWLGVIYSG
GSTDYNAASISRFTISKDNSKSTLYLQMNSLRAEDTAVYNCARNKVFQYYGSRYE
VMDYWGQGTLVTVSS
**SEQ ID NO: 26**
   **Anti-CCR8 antibody H-CDR2 (antibody E202h)**
   IYSGGSTD
**SEQ ID NO: 27**
   **Anti-CCR8 antibody H-CDR3 (antibody E202h)**
   CARNKVFQYYGSRYEVMDYW
**SEQ ID NO: 28**
   **Anti-CCR8 antibody VL sequence (antibody E202h)** 
DIQLTQSPSSLSASVGDRVTITCSASSSVVYMNWYQQKPGKAPKIWIYGISNLASG
VPSRFSGSGSGTDFTLTISSLQPEDFATYYCQQRTSSPPWTFGQGTKVEIK
**SEQ ID NO: 29**
   **Anti-CCR8 antibody VH sequence (antibody E203)** 
EVQLVETGGRLVQPKGSLKLSCAASGFTFNTNAMNWVRLAPGRGLEWVARIRSK
SNFYATHYADSVKDRFTVSRDDAQRMIYLQMNNLKTEDTAIYYCVRGKETGQNF
YAMDYWGQGTSVTVSS
**SEQ ID NO: 30**
   **Anti-CCR8 antibody H-CDR2 (antibodies E203 and E203h)**
   IRSKSNFYAT
**SEQ ID NO: 31**
   **Anti-CCR8 antibody H-CDR3 (antibody E203)**
   CVRGKETGQNFYAMDYW
**SEQ ID NO: 32**
   **Anti-CCR8 antibody VL sequence (antibody E203)** 
DVVMTQAAPSVLVTPGESVSISCRSSKSLLHSNGNTYLYWFLQRPGQSPQLLIYRM
SNLASGVPDRFSGSGSGTVFTLRISRVEAEDVGVYYCMQHLEYPFTFGAGTKLEV
K
**SEQ ID NO: 33**
   **Anti-CCR8 antibody VH sequence (antibody E203h)** 
EVQLVESGGGLVQPGGSLKLSCAASGFTFNTNAMNWVRLASGKGLEWVARIRSK
SNFYATHYADSVKDRFTISRDDSQRTLYLQMNSLKTEDTAVYYCVRGKETGQQF
YAMDYWGQGTLVTVSS
**SEQ ID NO: 34**
   **H-CDR3 (antibody E203h)**
   CVRGKETGQQFYAMDYW
**SEQ ID NO: 35**
   **Anti-CCR8 antibody VL sequence (antibody E203h)** 
DIVMTQSPLSLPVTPGEPASISCRSSKSLLHSQGQTYLYWFLQKPGQSPQLLIYRLS
NLASGVPDRFSGSGSGTDFTLKISRVEAEDVGVYYCFQHLEYPFTFGGGTKVEIK
**SEQ ID NO: 36**
   **Anti-CCR8 antibody L-CDR1 (antibody E203h)**
   KSLLHSQGQTY
**SEQ ID NO: 37**
   **Anti-CCR8 antibody L-CDR3 (antibody E203h)**
   FQBLEYPFT
**SEQ ID NO: 38**
   **IL-2V5** 
APTSSTKETQEQLERLLRDLQLILKGINNPKNPKLTRMLTFKFYMPKKATELKHLQ
CLEEELKPLEEVLNLAQSKNFHLRPRDLINNINKIVLKLKGSETTFMCEYADETATI
VEFLKRWIAFCQKIISTLT
**SEQ ID NO: 39**
   **IL-2V6** 
APTSSTKETQEQLERLLRDLQLILKGINNYKNPKLTRMLTFKFYMPKQATSLKHLQ
CLEEELKPLEEVLNLAQSKNFHLEPRNLINNINKIVLKLKGSETTFMCEYADETATI
VEFLKRWIAFCQSIISRST
**SEQ ID NO: 40**
   **IL-2V1** 
APTSSTKETQEQLERLLRDLQLILKGINNYKNPKLTRMLTFKFYMPKKATELKHLQ
CLEEELKPLEEVLNLAQSKNFHLRPRDLINNINKIVLKLKGSETTFMCEYADETATI
VEFLKRWIAFCQKIISTLT
**SEQ ID NO: 41**
   **Linker**
   GGGGSGGGGSGGGGS

## Claims

1. A fusion protein comprising an anti-CCR8 antibody, or a fragment thereof, and at least one IL-2 variant which is a regulatory T cell (Treg)-specific IL-2 receptor antagonist;
wherein the N-terminus of the at least one IL-2 variant is fused to the C-terminus of the antibody heavy chain.

2. The fusion protein according to claim 1, which induces Treg apoptosis.

3. The fusion protein according to claims 1 or 2, which depletes selectively tumor-infiltrating Tregs and in particular tumor-associated Tregs.

4. The fusion protein according to any one of claims 1 to 3, wherein the regulatory T cell (Treg)-specific IL-2 receptor antagonist comprises at least one, and in particular one, amino acid deletion at a position selected from S4, S5 or S6, the indicated positions being determined by alignment with wild-type human IL-2 set forth as sequence SEQ ID NO: 1.

5. The fusion protein according to any one of claims 1 to 4, wherein the regulatory T cell (Treg)-specific IL-2 receptor antagonist comprising the substitutions K9E, L12E, H16R, L19R, M23L, N26K, S87N, V91K, E95K, N119K and T123A;
and further comprises:
(i) the substitutions S127K and Y31P; or
(ii) the substitutions K49Q, E52S, R81E, D84N, T131R and L132S; or
(iii) the substitution S127K;
the indicated positions being determined by alignment with wild-type human IL-2 set forth as sequence SEQ ID NO: 1.

6. The fusion protein according to any one of claims 1 to 5, wherein the regulatory T cell (Treg)-specific IL-2 receptor antagonist comprising the substitutions K9E, L12E, H16R, L19R, M23L, N26K, S87N, V91K, E95K, N119K and T123A;
and further comprises the substitutions S127K and Y31P.

7. The fusion protein according to any one of claims 1 to 6, wherein the anti-CCR8 antibody comprises:
(i) a heavy chain comprising:
- a H-CDR1 having a sequence selected from the group consisting of the sequences set forth as SEQ ID NO: 3 and SEQ ID NO: 18;
- a H-CDR2 having a sequence selected from the group consisting of the sequences set forth as SEQ ID NO: 4, SEQ ID NO: 11, SEQ ID NO: 19, SEQ ID NO: 26 and SEQ ID NO: 30; and
- a H-CDR3 having a sequence selected from the group consisting of the sequences set forth as SEQ ID NO: 5, SEQ ID NO: 12, SEQ ID NO: 20, SEQ ID NO: 27, SEQ ID NO: 31 and SEQ ID NO: 34;
and
(ii) a light chain comprising:
- a L-CDR1 having a sequence selected from the group consisting of the sequences set forth as SEQ ID NO: 7, SEQ ID NO: 14, SEQ ID NO: 22 and SEQ ID NO: 36;
- a L-CDR2 having a sequence selected from the group consisting of the sequences set forth as SEQ ID NO: 8, SEQ ID NO: 15 and SEQ ID NO: 23; and
- a L-CDR3 having a sequence selected from the group consisting of the sequences set forth as SEQ ID NO: 9, SEQ ID NO: 16, SEQ ID NO: 24 and SEQ ID NO: 37;
or variants thereof having one or more conservative substitutions in one or more of these CDRs.

8. The fusion protein according to any one of claims 1 to 7, wherein the anti-CCR8 antibody comprises:
(i) a heavy chain comprising:
- a H-CDR1 having the sequence set forth as SEQ ID NO: 3;
- a H-CDR2 having a sequence selected from the group consisting of the sequences set forth as SEQ ID NO: 4 and SEQ ID NO: 11; and
- a H-CDR3 having a sequence selected from the group consisting of the sequences set forth as SEQ ID NO: 5 and SEQ ID NO: 12;
and a light chain comprising:
- a L-CDR1 having a sequence selected from the group consisting of the sequences set forth as SEQ ID NO: 7 and SEQ ID NO: 14;
- a L-CDR2 having a sequence selected from the group consisting of the sequences set forth as SEQ ID NO: 8 and SEQ ID NO: 15; and
- a L-CDR3 having a sequence selected from the group consisting of the sequences set forth as SEQ ID NO: 9 and SEQ ID NO: 16;
or
(ii) a heavy chain comprising:
- a H-CDR1 having the sequence set forth as SEQ ID NO: 18 ;
- a H-CDR2 having a sequence selected from the group consisting of the sequences set forth as SEQ ID NO: 19 and SEQ ID NO: 26; and
- a H-CDR3 having a sequence selected from the group consisting of the sequences set forth as SEQ ID NO: 20 and SEQ ID NO: 27;
and
a light chain comprising:
- a L-CDR1 having the sequence set forth as SEQ ID NO: 22;
- a L-CDR2 having the sequence set forth as SEQ ID NO: 23; and
- a L-CDR3 having the sequence set forth as SEQ ID NO: 24;
or
(iii) a heavy chain comprising:
- a H-CDR1 having the sequence set forth as SEQ ID NO: 3;
- a H-CDR2 having the sequence set forth as SEQ ID NO: 30; and
- a H-CDR3 having a sequence selected from the group consisting of the sequences set forth as SEQ ID NO: 31 and SEQ ID NO: 34;
and
a light chain comprising:
- a L-CDR1 having a sequence selected from the group consisting of the sequences set forth as SEQ ID NO: 7 and SEQ ID NO: 36;
- a L-CDR2 having a sequence selected from the group consisting of the sequences set forth as SEQ ID NO: 8 and SEQ ID NO: 15; and
- a L-CDR3 having a sequence selected from the group consisting of the sequences set forth as SEQ ID NO: 9 and SEQ ID NO: 37;
or variants thereof having one or more conservative substitutions in one or more of these CDRs.

9. The fusion protein according to any one of claims 1 to 8, wherein the anti-CCR8 antibody comprises:
(i) a heavy chain comprising:
- a H-CDR1 having the sequence set forth as SEQ ID NO: 3;
- a H-CDR2 having a sequence selected from the group consisting of the sequences set forth as SEQ ID NO: 4 and SEQ ID NO: 11, and is in particular SEQ ID NO: 11; and
- a H-CDR3 having a sequence selected from the group consisting of the sequences set forth as SEQ ID NO: 5 and SEQ ID NO: 12, and is in particular SEQ ID NO: 12;
and
(ii) a light chain comprising:
- a L-CDR1 having a sequence selected from the group consisting of the sequences set forth as SEQ ID NO: 7 and SEQ ID NO: 14, and is in particular SEQ ID NO: 14;
- a L-CDR2 having a sequence selected from the group consisting of the sequences set forth as SEQ ID NO: 8 and SEQ ID NO: 15, and is in particular SEQ ID NO: 15; and
- a L-CDR3 having a sequence selected from the group consisting of the sequences set forth as SEQ ID NO: 9 and SEQ ID NO: 16, and is in particular SEQ ID NO: 16;
or variants thereof having one or more conservative substitutions in one or more of these CDRs.

10. The fusion protein according to any one of claims 1 to 9, wherein:
(i) the variable domain of the heavy chain of the anti-CCR8 antibody, or fragment thereof, comprises, and in particular consists in, an amino acid sequence selected from the group consisting of the sequences set forth as SEQ ID NO: 2, SEQ ID NO: 10, SEQ ID NO: 17, SEQ ID NO: 25, SEQ ID NO: 29 and SEQ ID NO: 33;
and
(ii) the variable domain of the light chain of the anti-CCR8 antibody, or fragment thereof, comprises, and in particular consists in, an amino acid sequence selected from the group consisting of the sequences set forth as SEQ ID NO: 6, SEQ ID NO: 13, SEQ ID NO: 21, SEQ ID NO: 28, SEQ ID NO: 32 and SEQ ID NO: 35;
or variants thereof having one or more conservative substitutions in one or more of these sequences;
and in particular wherein :
(i) the variable domain of the heavy chain of the anti-CCR8 antibody, or fragment thereof, comprises, and in particular consists in, an amino acid sequence selected from the group consisting of the sequences set forth as SEQ ID NO: 2 and SEQ ID NO: 10, and is more particularly SEQ ID NO: 10;
and
(ii) the variable domain of the light chain of the anti-CCR8 antibody, or fragment thereof, comprises, and in particular consists in, an amino acid sequence selected from the group consisting of the sequences set forth as SEQ ID NO: 6 and SEQ ID NO: 13, and is more particularly SEQ ID NO: 13,
or variants thereof having one or more conservative substitutions in one or more of these sequences.

11. The fusion protein according to any one of claims 1 to 10, wherein the anti-CCR8 antibody fragment is selected from the group consisting of a Fv, Fab, F(ab')2, Fab', dsFv, scFv, sc(Fv)2 and a diabody.

12. The fusion protein according to any one of claims 1 to 11, wherein the anti-CCR8 antibody is devoid of fucosyl residues.

13. The fusion protein according to any one of claims 1 to 12, wherein the IL-2 variant has at least 85% sequence identity, in particular at least 90% sequence identity, more particularly at least 95% sequence identity, with any one of SEQ ID NO: 38, SEQ ID NO: 39 and SEQ ID NO: 40.

14. The fusion protein according to any one of claims 1 to 13, which further comprises a linker between the antibody heavy chain C-terminus and the IL-2 variant N-terminus, preferably a linker comprising, in particular consisting in, the sequence set forth as SEQ ID NO: 41.

15. A pharmaceutical composition comprising, in a pharmaceutically acceptable medium, at least a fusion protein according to any one of claims 1 to 14.

16. The fusion protein according to any one of claims 1 to 14, or the pharmaceutical composition according to claim 15, for use in the treatment of cancer.

17. The fusion protein for use or the pharmaceutical composition for use according to claim 16, wherein the cancer is selected from the group consisting of Melanoma; renal cancer, in particular Renal Cell Carcinoma; Colorectal Cancer; liver cancer, in particular Hepatocellular Carcinoma; lung cancer, in particular Non-Small Cell Lung Cancer; mesothelioma, in particular Malignant Pleural Mesothelioma; Esophageal Cancer, in particular esophageal squamous cell carcinoma or adenocarcinoma; Head and Neck cancer, in particular Head and Neck Squamous Cell Carcinoma; urothelial cancer, in particular urothelial carcinoma; Lymphoma, in particular Primary Hodgkin Lymphoma or Large B Cell Lymphoma; Gastric Cancer, in particular gastric or gastroesophageal junction adenocarcinoma; Cervical Cancer; Merkel Cell Carcinoma; Endometrial cancer, in particular endometrial Carcinoma; skin cancer, in particular cutaneous squamous cell carcinoma; Breast cancer, in particular Triple Negative Breast Cancer or Breast invasive Carcinoma; Pancreatic cancer, in particular pancreatic Adenocarcinoma; Thymoma; Prostate cancer, in particular prostate adenocarcinoma; Ovarian cancer, in particular ovarian serous cystadenocarcinoma; Thyroid cancer, in particular thyroid carcinoma, and Sarcoma.

18. The fusion protein for use or the pharmaceutical composition for use according to claim 16 or 17, in combination with at least one immune check-point inhibitor; in particular at least one selected from the group consisting of anti-PD-1, anti-PDL-1 or anti-CTLA-4 monoclonal antibodies.
